# EUROPEAN PATENT SPECIFICATION

(11) **EP 3 359 714 B1**
(45) Date of publication and mention of the grant of the patent: **17.03.2021**
(21) Application number: 16810227.5
(22) Date of filing: 07.10.2016
(51) Int. Cl.: A61L 27/20, D01D 5/34, D01D 5/06, D01F 8/04

(54) **ENDLESS CORE-SHEATH FIBERS ON THE BASIS OF HYALURONAN OR C11-C18 ACYLATED DERIVATIVES THEREOF, METHOD OF PREPARATION AND USE THEREOF, STAPLE FIBERS, YARN AND TEXTILES MADE OF THESE FIBERS AND USE THEREOF**
ENDLOS KERN-MANTEL-FASERN, ENTHALTEND EINE HYALURONAN ODER C11-C18 ACYLIERTE DERIVATEN DAVON, VERFAHREN ZU HERSTELLUNG UND VERWENDUNG, STAPELFASERN, GARN UND TEXTILIEN AUS DIESEN FASERN UND DEREN VERWENDUNG
FIBRES CONTINUES ÂME-GAINE SUR LA BASE D'HYALURONANE OU C11-C18 ACYLÉS DÉRIVÉS, PROCÉDÉ DE PRÉPARATION ET UTILISATION, FIBRES DISCONTINUES, FILS ET TEXTILES FABRIQUÉS DE CES FIBRES ET LEUR UTILISATION

(30) Priority: 09.10.2015 CZ 20150710
(43) Date of publication of application: 15.08.2018
(73) Proprietor: Contipro a.s., 56102 Dolni Dobrouc (CZ)
(72) Inventor: PITUCHA, Tomas, 53701 Chrudim (CZ); BETAK, Jiri, 56961 Dolni Ujezd (CZ); KUBICKOVA, Jolana, 56102 Dolni Dobrouc (CZ); KOCOVA, Sarka, 50312 Vsestary (CZ); JANOUCHOVA, Katerina, 27801 Kralupy nad Vltavou (CZ); RICHTROVA, Helena, 56401 Zamberk (CZ); LIPENSKA, Kristýna, 56151 Letohrad (CZ); ZAPOTOCKY, Vojtech, 27401 Slany (CZ); VELEBNY, Vladimir, 56401 Zamberk (CZ)
(74) Representative: Dvorakova, Martina
(86) International application number: PCT/CZ2016/050036
(87) International publication number: WO 2017/059834

(56) References cited:
- WO-A1-2014/082611
- CN-A- 103 789 874
- KR-A- 20080 111 815
- KR-A- 20120 118 681

## Description

### Field of the Invention

Present invention relates to biodegradable endless core-sheath fibers comprising a combination of native and C₁₁-C₁₈ acylated hylauronan or C₁₁-C₁₈ acylated hyaluronans and the method of preparation thereof. Preparation of the fibers is performed using wet spinning to give endless monofilaments with the structure core-sheath, followed by monofilaments processing with routine textile techniques into the form of linear textiles (yarns or braided threads), flat textiles (woven textiles, knitted textiles, braided textiles, nonwoven textiles) and tubular structures (braided, woven, knitted).

Prepared biodegradable fibers comprise two components - the core and the sheath that may have two utterly different functions, at the same time, they can carry utterly different active agents, which gives them advantages in applications to ordinary simple fibers. Applicable use of these fibers is directed to medicine, especially to the area of surgery materials, tissue engineering and systems for controlled release.

### Background of the invention

A possible technology for processing of hyaluronic acid into the form of a fiber is a method of wet spinning that was described for hyaluronic acid in the international application of invention WO 2009/050389. The fibers are obtained by coagulation in the bath of concentrated acetic acid (80% to 90%) followed by drying and drawing. Fibers prepared from native hyaluronan are very well water soluble, which is however disadvantageous for many applications. To reduce the solubility of fibers the authors state that the fiber can be coated using hydrophobic agents of plant or animal origin.

Also patent document WO2013167098 (A2) uses acetic acid (1 to 99%) for fiber preparation, although in the mixture with alcohol (1 to 99%) that adds higher strength to fibers.

Patents CZ 304303 and CZ 304266 use an analogous technology and coagulating baths (organic acid + lower alcohol) needed to obtain fibers on the basis of hyaluronic acid. However, they still obtain just simple fibers that do not have the core-sheath arrangement and they are not suitable for use for controlled release of active agent, as they do not enable releasing of native HA and optionally other active compounds. Moreover, in CZ 304266 are described fibers prepared from hyaluronan oxidized in the position 6 on N-acetyl-D-glucosamine part, it is therefore a derivative with different properties. Although these fibers show longer stability in wet environment than in the case of fibers from native hyaluronan, however, only for a period of about 30 minutes, after that they lose the fiber form and turn into gel. The fibers are not primarily determined as carriers of active agents.

In the description of patent CZ304303 are described simple fibers and textiles on the basis of hydrophobized HA. Although these fibers swell, they do not enable direct and controlled release of native HA, or of an active agent in the site of application.

Patent documents WO 93/11803, WO 98/08876, US 5658582, US 2004/0192643 describe formation of fibers and nonwoven textiles from esters of hyaluronic acid. The technological process of spinning used enables the formation of short fibers, though not formation of endless filament. The length of the fibers varies in the range of 5 to 100 mm and they can be processed only into the form of nonwoven textile.

In the term of applications of the fibers prepared from esters of hyaluronan, short fibers in the form of nonwoven textile are described (US 5658582), short fibers are also used for preparation of gauze, suture materials, nets, nonwoven textiles (WO 98/08876, US 006632802), or also for preparation of wound dressing material (WO 2007/003905).

Patent document WO 2010028025 describes fibers of hyaluronic acid and derivatives thereof (particularly salts), methods for preparation thereof and options of use (wrinkles filling, controlled release of drugs, surgery). The fiber is obtained by crosslinking of hyaluronic acid or derivatives thereof using 1,4-butanediol diglycidyl ether (BDDE). They are again only simple fibers.

Following patent documents relate to preparation of core-sheath fibers prepared using method of wet spinning.

US patent 8062654 describes gel or hydrogel core-sheath fibers. The basis are two polymer materials, where the polymer of the core is polymer gel with the potential content of cationic crosslinking agent. The other biodegradable material forming the sheath is well soluble in chlorinated solvents miscible with aliphatic solvents (pentane, hexane), that are used as coagulating (spinning) bath. The sheath of the fiber is made of polymer of lactic acid, polycaprolactone, polyglycolic acid or copolymers and mixtures thereof. The core of the fiber is made of alginate, crosslinked with addition of CaCO₃.

Patent document US 2012/0040463 describes preparation of hollow/multi-membrane fibers from fiber-forming solutions of polysaccharides or collagen using the wet spinning technology. The principle of preparation lies in repeated layering and coagulation of polymer material to fiber. It is stated in the description of the document, that the technology of cyclic coagulation is suitable for materials, that are transformable into the shape of endless fibers with difficulty, such as chitosan, hyaluronic acid, alginates, carboxymethyl cellulose, collagen.

Patent document US6162537 describes bicomponent fibers, wherein one component is of resorbable polymer and the other component is made of material that is able to form fibers and is not resorbable. It relates to polyesters, polyamides, polyolefins and polyurethanes, particularly polypropylene (PP), polyethylene (PE), polyhexamethylene terephtalate (PBTP). At least one component contains pharmacologically active ingredient. Use of the material is aimed at the area of nondegradable implantable medical devices. This implant is however determined only for applications, where it is not necessary to demand its complete degradation and resorption.

The most frequently described procedure of the preparation of the core-sheath fibers is spinning of melts, a technology called melt spinning. In this way the core-sheath fibers were prepared also in the US patent no. 8129449 or document US 2011/0028062. The melt spinning technology enables though only spinning of polymers that are meltable, which cannot be used in the case of materials that are the subject of present invention.

Patent document WO 2004/061171 describes fibers of the type "side by side", where there are 2 polymer materials of different properties but due to the arrangement of the two components in the fiber it is not possible to use the structure in cases, when the active agent, dispersed in one polymer component, is necessary to be covered with protecting sheathing layer.

Patent document WO 2006/102374 describes healing nettings of bicomponent core-sheath fibers where the bioactive agent is contained exclusively in the sheath. Bicomponent fiber contains non absorbing core and absorbent surface. Polymer material of the sheath is chosen from a group of polylactides, polyglycolides, p-dioxanes, poly(trimethylene carbonates), polycaprolactones, polyorthoesters. Polymer material suitable for the core is chosen from a group of polyesters, polyolefines, polyamides and fluorated polymers. A disadvantage of these fibers, or nets, is the content of bioactive agent exclusively in the sheath of the fiber, which excludes their protection and controlled release.

### Summary of the invention

Deficiencies resulting from the state of the art are solved by the endless core-sheath fiber based on hyaluronan or C₁₁-C₁₈ acylated derivative thereof, the subject-matter of which is that it comprises a combination of hyaluronan and acylated derivative thereof or a combination of acylated derivatives thereof, wherein the core and sheath of the fiber are in any of the following arrangements:
A) the core contains hyaluronic acid or a salt thereof, the sheath contains C₁₁-C₁₈ acylated hyaluronan;
B) the core contains C₁₁-C₁₈ acylated hyaluronan, the sheath contains hyaluronic acid or a salt thereof;
C) the core contains C₁₁-C₁₈ acylated hyaluronan, the sheath contains different C₁₁-C₁₈ acylated hyaluronan differing in C₁₁-C₁₈ acyl from C₁₁-C₁₈ acylated hyaluronan of the core, whereas the degree of substitution of C₁₁-C₁₈ acylated hyaluronan in the sheath is the same or different from the degree of substitution of C₁₁-C₁₈ acylated hyaluronan of the core; or
D) the core contains C₁₁-C₁₈ acylated hyaluronan, the sheath contains different C₁₁-C₁₈ acylated hyaluronan differing in the degree of substitution from C₁₁-C₁₈ acylated hyaluronan of the core.

Options of core-sheath arrangement components in terms of their material constitution are apparent from Table 1.

**Table 1. Options of the arrangement of the core-sheath fiber components of the invention.**

| Core | Sheath |
|---|---|
| Native hyaluronan | C₁₁-C₁₈ acylated hyaluronan |
| C₁₁-C₁₈ acylated hyaluronan | Native hyaluronan |
| C₁₁-C₁₈ acylated hyaluronan | C₁₁-C₁₈ acylated hyaluronan - acylated using fat acid different from that one of the core |
| C₁₁-C₁₈ acylated hyaluronan | C₁₁-C₁₈ acylated hyaluronan-degree of substitution different from that one of the core |

The fiber of the invention comprises two components, the core containing hyaluronic acid or a salt thereof or C₁₁-C₁₈ acylated hyaluronan and the sheath containing hyaluronic acid or a salt thereof or C₁₁-C₁₈ acylated hyaluronan. If both the fiber core and the fiber sheath of the invention contain C₁₁-C₁₈ acylated hyaluronan, then the C₁₁-C₁₈ acylated hyaluronans differ in chemical structure. The difference consists in either different kind of C₁₁-C₁₈ substituent, and/or different degree of substitution.

For the fiber of the invention of arrangement C applies, that the sheath contains different C₁₁-C₁₈ acylated hyaluronan differing in C₁₁-C₁₈ acyl from C₁₁-C₁₈ acylated hyaluronan of the core, whereas its degree of substitution is the same or different compared with the degree of substitution of C₁₁-C₁₈ acylated hyaluronan of the core.

For the fiber of the invention of arrangement D applies, that the sheath contains different C₁₁-C₁₈ acylated hyaluronan differing in the degree of substitution from C₁₁-C₁₈ acylated hyaluronan of the core, whereas the kind of C₁₁-C₁₈ substituent is the same.

C₁₁-C₁₈ acylated hyaluronan is preferably acylated in primary alcohol of *N*-acetyl-glucosamine.

Molar mass of C₁₁-C₁₈ acylated hyaluronan in the core and/or sheath in the above fiber arrangements of the invention is in the range 1x10⁵ to 7x10⁵ g/mol, preferably 2x10⁵ to 3x10⁵ g/mol.

According to the preferred embodiment of the invention C₁₁-C₁₈ acylated hyaluronan is chosen from a group containing of palmitoyl hyaluronan, stearoyl hyaluronan or oleoyl hyaluronan.

According to the preferred embodiment of the invention the molar mass of hyaluronic acid and/or the salt thereof is in the range 1x10⁵ to 2x10⁶ g/mol, preferably 8x10⁵ to 1.8x10⁶ g/mol, whereas the salt of hyaluronic acid is chosen from a group comprising alkali metal ions, alkaline earth metal ions, preferably Na⁺, K⁺, Ca²⁺ or Mg²⁺.

According to another preferable embodiment of present invention the degree of substitution of C₁₁-C₁₈ acylated hyaluronan in the arrangements A or B is in the range 5 to 80 %, preferably 30 to 60 %. Further, the degree of substitution of C₁₁-C₁₈ acylated hyaluronan contained in the core of arrangement C is in the range 5 to 80 %, preferably 5 to 29 %, more preferably 10 to 20 % and the degree of substitution of C₁₁-C₁₈ acylated hyaluronan contained in arrangement C is in the range 5 to 80 %, preferably 30 to 80 %, more preferably 40 to 60 %. Further, the degree of substitution of C₁₁-C₁₈ acylated hyaluronan contained in the core of arrangement D is in the range 5 to 29 %, preferably 10 to 20 % and the degree of substitution of C₁₁-C₁₈ acylated hyaluronan contained in the sheath of arrangement D is in the range 30 to 80 %, preferably 40 to 60 %.

Both fiber components of the invention can preferably contain at least one active agent. According to the preferable embodiment of the fibers of the invention, the core and sheath contain both at least one identical or different active agent. The fiber may then contain simultaneously utterly different active agents that may be released with different rate depending on their placement in fiber components of the invention. The active agent is selected from a group comprising antibacterial, antiseptic, antibiotic, anti-inflammatory, hemostatic, anesthetic, cytostatic, hormones, immunomodulators, immunosuppressives or agents for contrast imagining, preferably magnetic nanoparticles or fluorescent agents. Active agents may be also incorporated into micelles and through them into fiber core.

In combination of native HA and acylated HA the fiber is arranged either with the core being made of native hyaluronan and the sheath of C₁₁-C₁₈ acylated hyaluronan or in the reverse arrangement, when the core is made of C₁₁-C₁₈ acylated hyaluronan and the sheath of native hyaluronan, then it is referred to as "reverse fiber". In the fiber, there is then combined water soluble component (native HA) with the component of hydrophobic nature (acylated HA), that shows decreased solubility, or in other words higher stability in aqueous environment.

If the fiber core of the invention is made of native hyaluronan and the fiber sheath of acylated hyaluronan, then the fiber sheath fills the protective function related to the fiber core according to the invention. The sheath ensures mechanical protection of the core during textile processing and also the protection from aggressive environment that could lead to disruption of the fiber and premature or too fast release of native hyaluronan, eventually active agent from the fiber core of the invention.

In the case of reverse arrangement of the fiber of the invention acts the fiber core containing acylated hyaluronan as a reinforcing component that ensures mechanical cohesiveness of the fiber, eventually of manufactured textile, also after the contact with liquid, e.g. water or body fluids.

Another option of fiber arrangement is a combination of two hyaluronan derivatives acylated using two different C₁₁-C₁₈ fatty acids on hydroxyl groups of hyaluronan. The fiber core is preferably made of oleyl hyaluronan and the fiber sheath of palmitoyl hyaluronan.

Still another option of fiber arrangement is a combination of two hyaluronan derivatives acylated using the same C₁₁-C₁₈ fatty acid on hydroxyl groups of hyaluronan, but differing in degree of substitution. The fiber core is preferably made of palmitoyl hyaluronan with degree of substitution 5 to 29 % and the fiber sheath of palmitoyl hyaluronan with degree of substitution of 30 to 80 %. In this preferred arrangement provides the hydrophobic sheath mechanical protection of the core during textile processing and also the protection from aggressive environment, whereas the core that is thanks to low degree of substitution only mildly hydrophobic, may carry an active agent.

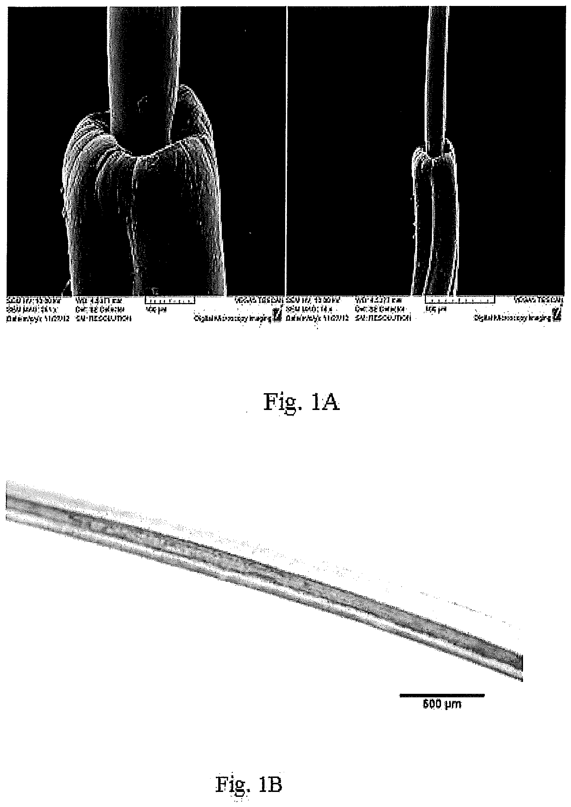

According to preferred embodiment of the invention the fiber fineness is in the range 10 to 40 tex, preferably 15 to 35 tex, more preferably 22 to 28 tex and the volume ratio core:sheath is in the range 3:1 to 1:6, preferably 1:3 to 1:5, more preferably 1:4. The cross-section of the fiber of the invention has irregular shape, typical for fibers made using the method of wet spinning (see Fig. 1A). The value of equivalent fiber intersection for various fiber fineness is in the Table 2. Tensile strength of the fibers is in the range 0.8 to 3.5 N, breaking elongation is in the range 8 to 30 %.

**Table 2. Values of equivalent diameter of the fiber of the invention for various fineness values.**

| Fiber fineness [tex] | Equivalent diameter [µm] |
|---|---|
| 10 | 90 |
| 15 | 110 |
| 20 | 130 |
| 25 | 145 |
| 30 | 160 |
| 35 | 170 |
| 40 | 180 |

The fiber of the invention is preferably used for production of yarns, braided, woven or knitted textiles.

Applicable use of the core-sheath fibers of the invention is directed to medicine, particularly to the area of surgery materials, tissue engineering and systems for controlled release of active agents.

Component arrangement in the fibers of the invention is preferably centric, as is shown in Fig. 1A, when the fiber core is placed in the center of the sheath. In Fig. 1A for illustration there is shown fiber of the invention with exposed core, when in one section of the fiber the sheath does not cover the core. However, the fibers prepared according to the method of the invention provide monofilaments, i.e. endless core-sheath fibers of the invention, where the sheath covers the core throughout the length.

The core-sheath fibers of the invention are prepared using the technology of wet spinning. It is the technology that is gentle to polymers that degrade at high temperature. At the same time it is possible to implement the active agent that is at high temperature unstable, undergoes decomposition or degradation into polymer solutions spun using this technology, and obtain the fiber that contains this active agent. The disadvantage of the active agent incorporation into simple fibers may be the elution of the active agent through diffusion processes during fiber coagulation. This disadvantage may be attenuated or stopped just by the core-sheath structure wherein the sheath ensures barrier function, avoiding active compound elution into coagulation bath.

The method of preparation of core-sheath fibers of the invention is in separated preparation of two different spinning solutions of polymers on the basis of hyaluronan followed by their simultaneous spinning using coaxial spinneret.

In one embodiment of the method of the invention for fiber spinning of the arrangement A or B there is separately prepared the first spinning solution of native hyaluronan in concentration range 1 to 8 wt% in demineralized water and the second spinning solution of C₁₁-C₁₈ acylated hyaluronan in concentration range 2 to 8 wt% in the mixture of lower alcohol and demineralized water, where the water content is in the range 30 v/v% to 90 v/v% and the lower alcohol content is in the range 10 v/v% to 70 v/v%, followed by extrusion of the first and the second spinning solution together into coagulation bath containing 2 to 40 wt% of organic acid, preferably lactic acid, at least 50 wt% of lower alcohol, preferably it is used ethanol, propan-1-ol or propan-2-ol and 2 to 48 wt% of water resulting in fiber formation of arrangement A or B, that are further scoured with lower alcohol and dried.

In another embodiment of the method of the invention for fiber spinning of arrangement C or D there is separately prepared the first spinning solution containing C₁₁-C₁₈ acylated hyaluronan of concentration range 2 to 8 wt% in the mixture of water and the lower alcohol and the second spun solution containing different C₁₁-C₁₈ acylated hyaluronan of concentration 2 to 8 wt% in the mixture of water and lower alcohol, where water content in both solutions is in the range of 30 v/v% to 90 v/v%, lower alcohol content is in the range 10 v/v% to 70 v/v%, followed by extrusion of the first and the second solution together into coagulation bath containing 2 to 40 wt% of organic acid, preferably lactic acid, at least 50 wt% of lower alcohol, preferably is used ethanol, propan-1-ol, or propan-2-ol and 2 to 48 wt% of water resulting in fiber formation of arrangements C or D, that are further scoured with lower alcohol and dried.

Preferably it is performed the method of the invention, whereas the first spun solution and/or the second spun solution contains the active agent or the nanomicellar composition based on acylated hyaluronan containing active agent.

In terms of molar mass are suitable fractions of native hyaluronan in the range 1x10⁵ to 2x10⁶ g/mol. Used fraction of molar masses of acylated hyaluronan is in the range 1x10⁵ to 7x10⁵ g/mol, preferably 2x10⁵ to 3x10⁵ g/mol. The degree of substitution (acylation) of C₁₁-C₁₈ acylated hyaluronan for fiber arrangement A or B of the invention is in the range 5 to 80 %, preferably 30 to 60 %. The degree of substitution of C₁₁-C₁₈ acylated hyaluronan contained in the core of arrangement C is further in the range 5 to 80 %, preferably 5 to 29 %, more preferably 10 to 20 % and the degree of substitution of C₁₁-C₁₈ acylated hyaluronan contained in the sheath in arrangement C is in the range 5 to 80 %, preferably in the range 30 to 80 %, more preferably 40 to 60 %. The degree of substitution of C₁₁-C₁₈ acylated hyaluronan contained in the core of arrangement D is further in the range 5 to 29 %, preferably 10 to 20 % and the degree of substitution of C₁₁-C₁₈ of acylated hyaluronan contained in the sheath of arrangement D is in the range 30 to 80 %, preferably in the range 40 to 60 %.

Low molecular weight alcohol for the first and/or the second spun solution is preferably chosen from a group comprising methanol, ethanol, propan-1-ol, or propan-2-ol.

Prepared spun solutions are extruded together through a two-way coaxially arranged spinneret into coagulation bath, as is described above. The coaxial spinneret is composed of inner spinneret of circular cross-section and outer spinneret with the cross-section in the shape of annulus. When the core-sheath fiber of the invention is formed, the spinning solution forming the fiber core of the invention is extruded through the inner spinneret and the spinning solution forming the fiber sheath of the invention is extruded through the outer spinneret. Obtained fiber is further scoured in a bath of lower alcohol, preferably ethanol, propan-1-ol or propan-2-ol, and freely air-dried or dried in a vacuum drier at mildly elevated temperature (up to 40 °C). The described method of the fiber preparation and the range of the spinning conditions apply for all variants of component arrangement within the core-sheath fiber of the invention. Prepared core-sheath fibers of the invention may be further processed by common textile techniques into the shape of linear textiles (twisted yarns or braided threads), flat textiles (woven textiles, knitted textiles, braided textiles, nonwoven textiles) and tubular structures (braided, woven, knitted).

Above described method of the invention may be used for production of fibers, the core thereof is made of native hyaluronan and the sheath of acylated hyaluronan, or also "reverse" fibers, wherein the core is made of acylated hyaluronan and the sheath of native hyaluronan. Reverse fibers are characterized in that hyaluronan sheath of the fiber, when in contact with wet environment, transforms into biocompatible gel, that then increases bioacceptance of the whole fiber conjugate and at the same time eventually slows down native hyaluronan release, or active agent release in the site of application, when it is contained in the sheath. If such fibers are processed into the form of a textile, than after its contact with aqueous medium (e.g. water or body fluids) occurs formation of gel compact layer of hyaluronan that fills pores in the textile, while the core of acylated hyaluronan ensures mechanical cohesiveness of the textile. Thus it develops the closed composite textile structure filled with gel that can be used e. g. as anti-adhesive device that forms a barrier and prevents undesirable adhesions of tissues or organs.

The core- sheath fibers of the invention combine water-soluble and insoluble (swelling) part (depending on core and sheath composition), or two insoluble components, that can differ from each other in the extent of hydrophobicity and swelling, depending on used C₁₁-C₁₈ acylated hyaluronan and its degree of substitution. Fibers are biologically degradable, thus they are gradually degraded and absorbed in living organism.

The preferred range of geometric characteristics of fiber stated above partly depend on trouble-free process of spinning and partly on processability with textile technologies. For ensuring continuous fiber forming without ruptures there are more preferred increased values of fineness. For textile process, it is conversely better to use finer fibers, as they are more flexible but at the same time it is necessary to retain enough width of the sheath to prevent its rupture through friction by working elements of the machine. In preferred range of fineness and volume ratios core:sheath used, it is than ensured the satisfactory mechanical resistance of the fiber needed for textile processing

The fibers of the invention may be used for further textile processing either in the form of endless fiber, or in the form of staple fibers. Staple fibers may be prepared by cutting or tearing endless fibers of the invention to short sections of the length 3 to 150 mm, preferably 50 to 90 mm.

Endless fiber of the invention may be processed into the form of a yarn. Yarn may be made of one individual fiber in the form of non-twisted monofilament, or in the form of a bundle containing 2 to 10 fibers, preferably 3 to 6 such fibers. The fineness of the yarn may be in the range of 10 to 400 tex, preferably 30 to 100 tex. The yarn of the invention made of a bundle of fibers may preferably contain at least one twist per 1 meter of the length resulting in a twisted bundle.

Yarn, i.e. either single fiber (monofilament) of the invention or a bundle of these fibers collaterally oriented or a twisted bundle of fibers may be processed by braiding, knitting or weaving. Staple fibers may be processed into the form of nonwoven textile, preferably needle-punched or stitch-bonded textile. Braided, woven, knitted or non-woven textiles of the invention are applicable in the area of medicine, tissue engineering and systems for controlled release.

According to another embodiment of the invention is the yarn made of at least two fibers, whereas at least one fiber is the fiber of the invention, as defined above, and at least one fiber is the fiber selected from a group of fibers of other biodegradable materials, whereas biodegradable material is selected from a group comprising polylactic acid (PLA), polyglycolic acid (PGA), copolymer of polylactic and polyglycolic acid (PLGA), polycaprolactone (PCL), polydioxanone (PDO) or polyhydroxyalcanoates (PHA), preferably polylactic acid (PLA) or a copolymer of polylactic acid and polyglycolic acid (PLGA). It is further possible to use fibers of inert material, that are selected from a group comprising polyethylene (PE), polypropylene (PP), polyetherester (PEE), polyamide (PAD) or polyester (PES).

The fineness of yarn made of the fibers of the invention is in the range 10 to 400 tex, preferably 30 to 100 tex. It may be braided using technologies commonly used and known in the state of the art forming linear braided textiles (braided thread), flat (braided band), or tubular (braided tube), eventually textiles with non-circular, e. g. square-shaped cross-section. The character of textile structure depends on the method of fibers or yarns interlacing, i.e. on technological setting of the braiding process.

According to the preferred embodiment of the invention it may be manufactured the braided textile, that contains at least one yarn of the invention, whereas at least one fiber is of the invention in component arrangement A, B, C or D, as is defined above, or is made of yarns of the invention.

Braided textile may be made of 3 to 96 yarns of the invention. Processing on the flat braiding machine produces a narrow flat braided textile with a character of a band. Processing on a circular braiding machine produces a braided textile of an approximately circular cross-section that can by either compact, or tubular, i.e. has a hollow in the center. If the braided textile of 3 to 11 yarns of the invention, preferably of 3, 4, or 8 yarns is produced by circular braiding machine, it has a nature of a thread with compact cross-section. Braiding the textile of 12 to 96 yarns, preferably of yarns, the number thereof is a multiple of 4, more preferably of 12, 16, 24, 32 or 48 yarns, the braided textile with tubular structure, i.e. there is an inner hollow along the braided textile is produced by the circular braiding machine. At least one filling fiber may be inserted in this hollow during braiding using common technology. If the volume of the hollow is not entirely filled with filling fibers, the cross-section of the braided textile maintains tubular character. If the volume of the hollow is totally filled up with filling fibers, the cross-section of the braided textile adopts compact character. Filling fibers may be inserted alongside the thread, also in a textile braided from 8 threads, during the braiding on a circular braiding machine using a common technique. The cross-section of such braided textile has also a compact character.

Filling fibers may be in the form of yarn of the invention or of the yarn from staple fibers or in the form of braided thread. The diameter, or equivalent diameter of filling fiber is in the range 0.0001 to 1 mm, preferably 0.01 to 0.1 mm.

If it is desired to reach the "effect of rupture" of the sheath, as is described below, the cross-section of the braided textile is preferably compact.

Filling fibers may be made of biodegradable polymers chosen from a group comprising hyaluronic acid or derivatives thereof, polylactic acid (PLA), polyglycolic acid (PGA), copolymer of polylactic acid and polyglycolic acid (PLGA), polycaprolactone (PCL), polydioxanone (PDO), polyhydroxyalkanoates (PHA), preferably polylactic acid (PLA) or copolymer of polylactic acid and polyglycolic acid (PLGA). As filling fibers may be used fibers of inert material, thus nonresorbable biocompatible polymer, as is e. g. polyethylene (PE), polypropylene (PP), polyetherester (PEE), polyamide (PAD), polyester (PES) etc.

Another option of processing yarns of fibers of the invention is through weaving. Weaving is understood as producing woven flat textile from one or more lengthwise (warp) sets of yarns and from one or more crosswise (weft) sets of yarns mutually interwoven in perpendicular direction on weaving loom. Warp respectively weft yarn is understood as single fiber of the invention, bundle of such fibers oriented parallelly or twisted bundle of these fibers.

Yet another option of processing the yarns of fibers of the invention (single fibers, bundles of parallel or twisted fibers) is through knitting. Knitting is understood as manufacturing of knitted flat or tubular textile from yarns of the invention shaped into mutually intermeshed loops, optionally with the use of other knit elements, ordered into wales and courses. Knitted flat or tubular textile may be e.g. single-faced, double-faced or purl weft-knitted textile or single-faced or double-faced warp-knitted textile, preferably tricot-knit, twill-knit or satin-knit fabric or a knitted fabric with inlaid weft. Yarn is understood as a single fiber of the invention, a bundle of the fibers oriented parallelly or a twisted bundle of these fibers.

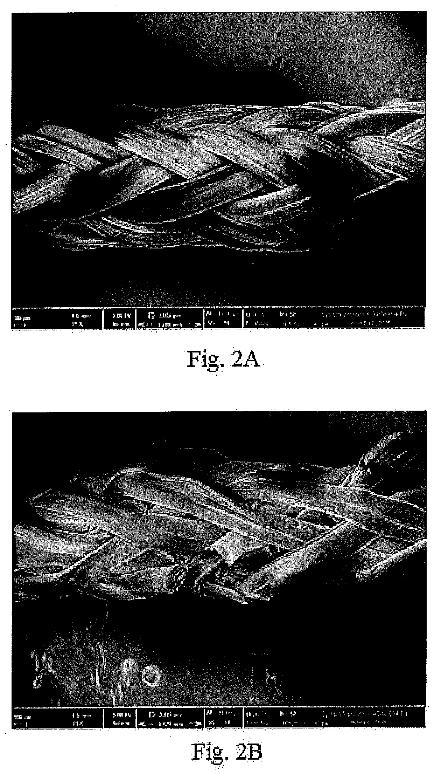

In linear, flat or tubular textiles produced from the fibers of the invention, where the core and sheath of the fiber in the yarn are in the arrangement A, C or D, while in the case of arrangement A is the degree of substitution of C₁₁-C₁₈ acylated hyaluronan in the sheath in the range 30 to 80 %, preferably 40 to 60 % and in the case of arrangements C or D the degree of substitution of C₁₁-C₁₈ acylated hyaluronan in the core is in the range 5 to 29 %, preferably 10 to 20 % and the degree of substitution of C₁₁-C₁₈ acylated hyaluronan in the sheath is in the range 30 to 80 %, preferably 40 to 60 %, may be used synergic effect of textile weave and swelling of fibers on the basis of HA for controlled release of active agent. In contact of the textile with liquid agent, e. g. water or body fluid occurs fiber swelling that results in mechanical stress in the fiber followed by rupture of the structure of fiber sheath of the kind core-sheath ("the effect of rupture"). Resulting ruptures enable controlled release of both native hyaluronan, eventually acylated hyaluronan with the degree of substitution 5 to 29 %, preferably 10 to 20 %, and also optionally of an active agent (e.g. pharmaceutical agent or inorganic particles), if it is contained in fiber core. This is shown in Fig. 2A and Fig. 2B. Fig. 2A shows initial braided textile of the invention from core-sheath fibers of the invention, Fig. 2B shows this textile after its wetting in water, core spilling and drying; well observable is the disrupted fiber sheath that shows local ruptures, while fibers have flattened structure resulting from spilling of soluble hyaluronan out from the core. In fibers of the invention, where the volume ratio core:sheath is in the range of cca 3:1 to 1:3 may at swelling come to the "effect of rupture" also in single non-braided/knitted/woven fiber, fiber bundle or twisted fiber bundle, because of small width of the sheath. Otherwise this effect only occurs when the fibers of the invention are processed into the form of braided, woven or knitted textiles, because at swelling pressure generation occurs in the textile structure and through this pressure is the fiber sheath of the invention torn. Surprisingly, it was found that at swelling of braided, woven or knitted textiles of the invention occurs only partial tearing of the sheath of single fibers and the textile structure remains, despite many interruptions, compact. No significant swelling up into incoherent gel occurs, neither occurs desintegration of textile structure into single fibers, but textile structure is maintained, as is apparent in Fig. 2B.

In the case of braided textiles occurs the effect of structure rupture at the angle of braiding at least 20°, preferably at least 25°, while there applies that if the fineness of the yarn is x tex, than braiding density of the yarn is at least y cm⁻¹, preferably at least z cm⁻¹, as is stated in Table 3 below.

**Table 3. Braiding density needed for reaching the "effect of rupture" for yarns of different fineness.**

| Yarn fineness [tex] x | Braiding density [cm⁻¹] y | Preferred area of braiding density [cm⁻¹] z |
|---|---|---|
| Less than 20 | 15 | 20 |
| 20 to 30 | 12 | 15 |
| 30 to 50 | 9 | 12 |
| 50 to 80 | 7 | 9 |
| 80 to 120 | 6 | 7 |
| 120 to 180 | 5 | 6 |
| 180 to 270 | 4 | 5 |
| More than 270 | 3 | 4 |

Braiding angle is understood as the angle that is formed between the yarn and the longitudinal axis of braided textile, or in other words, half angle between mutually interlaced yarns in respect to the direction of braiding.

Braiding density is understood as the number of consecutive transitions of yarns from face to back (in flat textile), or from outside to inside (in the case of tubular textile), per 1 cm of the braided textile length.

Upper limit of braiding angle is 45°, braiding density is limited by processability and limit geometry of braided structure.

Other parameters of the braided textile:
Tubular braided textile diameter or braided thread diameter: 0.3 to 15 mm
Flat braided textile width: 0.3 to 15 mm
Number of braided yarns in a textile: 3 to 96 yarns
Range of yarn fineness is 10 to 400 tex, preferably 30 to 100 tex.
Number of filling fibers forming the filling of tubular structure resulting from braiding of 8 and more yarns: 1 to 1000.
Diameter of fiber/fibers forming filling of tubular braided textile: 0.0001 to 1 mm, preferably 0.01 to 0.1 mm.

To ensure the "effect of rupture" of the fiber sheath of the invention in woven textile of the invention it is necessary to apply that if the yarn fineness is a tex, then fabric sett is at least b cm⁻¹, preferably at least c cm⁻¹, as is stated in Table 4 below. Fabric sett is understood as the number of warp or weft yarns per 1 cm width of woven textile. Preferably may be used plain weave or twill.

Upper limit of fabric sett is limited by yarn processability and limit geometry of woven structure.

The range of yarn fineness is 10 to 400 tex, preferably 30 to 100 tex.

**Table 4. Fabric sett needed to reaching of "effect of rupture" for yarns of various fineness**

| Yarn fineness [tex] a | Fabric sett [cm⁻¹] b | Preferable area of Fabric sett [cm⁻¹] c |
|---|---|---|
| Less than 20 | 25 | 35 |
| 20 to 30 | 18 | 25 |
| 30 to 50 | 14 | 20 |
| 50 to 80 | 11 | 16 |
| 80 to 120 | 9 | 13 |
| 120 to 180 | 7 | 11 |
| 180 to 270 | 6 | 9 |
| More than 270 | 5 | 7 |

To ensure the "rupture effect" of the fiber sheath of the invention in knitted textiles of the invention, it is necessary to apply that if the yarn fineness is e tex, then column density is at least f dm⁻¹, preferably at least g dm⁻¹, as it is stated in Table 5 below. Wale density is understood as the number of wales per 1 dm width of knitted textile.

Upper limit of wale density is limited by yarn processability and limit geometry of knitted structure.

The range of yarn fineness is 10 to 400 tex, preferably 30 to 100 tex.

**Table 5. Colum density of links of braided textile needed for the "effect of rupture" for yarns of different fineness.**

| Yarn fineness [tex] e | Wale density [dm⁻¹] f | Preferable area of wale density [dm⁻¹] g |
|---|---|---|
| Less than 20 | 20 | 27 |
| 20 to 30 | 18 | 23 |
| 30 to 50 | 16 | 20 |
| 50 to 100 | 14 | 18 |
| More than 270 | 12 | 16 |

In the case that during weaving or knitting is used braided linear, flat or tubular textile (thread, band, tube) of the invention with the parameters of textile with "effect of rupture", than it does not depend on the structure of woven or braided flat textile itself, as the rupture effect is secured by braided linear, flat or tubular textile of the invention with textile parameters of "effect of rupture" and therefore it would express also in woven or knitted textiles with open character of mesh.

### Controlled release

In textiles made of core-sheath fibers of the invention whrein the sheath thereof is made of acylated HA with the degree of substitution of 30 to 80 %, preferably 40 to 60 % and the core is made of native HA or acylated HA with the degree of substitution of 5 to 29 %, preferably 10 to 20 %, it is possible to control the course of core release, and through that also the release of active agent optionally contained in it. It is possible to control the beginning of release, rate of release, overall period of release, amount of released agent and in the case of flat and tubular textiles also the place of release. The process of release is initiated by contact of textile with a liquid, e. g. water, body fluid, or other liquid. If it is desired that the process starts as soon as possible after textile insertion in wet environment, it is necessary to use a very tight textile structure, in which the sheath starts to tear at low amount of sorbed liquid. In less tight structures is the beginning of release delayed to the while when the environment contains such amount of liquid that after absorption into fiber causes its full swelling. The rate of core release and total time of release depend on the amount of ruptures in the sheath. The bigger distance between two ruptures, the longer it takes for the core to "spill out" from the sheath. The amount of the ruptures, respectively their spacing may be specifically influenced by combination of closely and sparsely interlaced areas in the textile, i.e. for example by changing the braiding density in braided textiles or different stitch density in the area of knitted textile. Sheath rupture and core release then occurs only in tight places of the textile, and through these ruptures then gradually releases also the core from sparse segments. The rate of release may be also influenced by the core composition of the fiber of the invention. The core made of native HA releases faster, the core made of acylated HA with the degree of substitution 5 to 29 %, preferably 10 to 20 % releases more slowly. Total period of release may be in this way regulated in the range of tens of minutes to tens of hours. If there is the active agent comprised in the fiber core, then it releases together with the core. Total amount of the released active agent then depends on its concentration in the fiber core, the mass ratio of core-sheath and total amount of fibers in textile sample that fills the function of active agent carrier. Fibers of the invention may be in the scope of the textile variably spread (similarly like color pattern made of different yarns), and thus the effect of core, or contained active agent is targeted to the desired place, the treated tissue. The fibers may be concentrated into the specific area of textile pattern in the scope of the area (e.g. center, or peripheries) or laterally (back, or face).

As it was stated above, both core and sheath of the fiber of the invention may contain an active agent, e. g. a pharmaceutical ingredient or inorganic particles. The kind, concentration of active agent in the core and/or sheath of the fiber of the invention may differ depending on application of textile of the invention. The examples are introduced in Table 6:

**Table 6. Examples of active agents.**

| **Pharmaceutical present in fiber sheath** | **Pharmaceutical present in fiber core** |
|---|---|
| Anti-inflammation agent | Antipyretic |
| Hemostatic | Immunosuppressive drug |
| Antibiotic I | Antibiotic II |
| Antiseptic | Anti-inflammation agent |

The options of the use of the textiles of the invention with different kinetics of release:
Fast hemostatic release within a few hours may be used e.g. to reduce tissue bleeding during surgery or shortly thereafter.

Medium fast release of local anesthetic within several hours may be used to reduce pain e.g. in burns or after surgery.

Slow antiseptic release within 1 to 2 days may be used for dressings for infectious chronical wounds.

Slow fibrinolytic release within days may support the effect of antiadhesive agents.

Braided, woven, knitted textiles or non-woven textiles of the invention may be used in the area of medicine, tissue engineering and systems for controlled release.

### Definitions

The term "single fiber" means a fiber consisted of only one morphological component.

The term "lower alcohol" means C₁-C₆ alkanol.

The term "core-sheath fiber" means a two-component (bicomponent) fiber consisted of fiber core and sheath.

The term "degree of substitution" = 100 % ^{∗} molar amount of bound acyl/ molar amount of all polysaccharide dimers.

The term "equivalent fiber diameter" means diameter of a circle with an area identical to the area of cross-section of the fiber of the invention.

The term "hyaluronan" or "HA" or "native hyaluronan" means hyaluronic acid and/or a salt thereof processable or processed into the form of the core-sheath fiber of the invention.

The term "C₁₁-C₁₈ acylated hyaluronan" or "C₁₁-C₁₈ acylated HA" means hyaluronan acylated using C₁₁-C₁₈ fatty acid in hydroxyl groups of hyaluronan.

The term "co-extrude" means simultaneously extrude two different polymer solutions into coagulation bath through the two-way coaxial spinneret.

The term "monofilament" means the single endless fiber.

The term "yarn" is understood as the single endless fiber (monofilament), the bundle of parallel fibers or the twisted bundle of fibers.

The term "twisted bundle of fibers" means at least two parallel fibers forming a bundle, with at least one twist per 1 m.

The term "staple fibers" means a set of fibers made by cutting or tearing of endless fibers. The staple fibers may have different lengths (in the range 3 to 150 mm).

The term "fineness" expressed in [tex] units, means weight of 1 km of the fiber or yarn in grams (1 tex = 1 g/km).

The term "inert material" means the non-absorbable biocompatible polymer.

The term "effect of rupture" means disruption of the sheath containing acylated hyaluronan with a degree of substitution 30 to 80 %, preferably 40 to 60 %, in the core-sheath fiber resulting from mechanic stress produced by fiber swelling in textile structure and followed release of core made of native hyaluronan or acylated hyaluronan with degree of substitution 5 to 29 %, preferably 10 to 20 %.

The term "braiding angle" means the angle between the yarn and longitudinal axis of the braided textile, or half-angle between mutually interlaced yarns in respect to the direction of braiding.

The term "braiding density" means the number of consecutive yarn transitions from face to back (in flat textile), or from outside to inside (in tubular textile), per 1 cm of the length of the braided textile.

The term "fabric sett" means the number of warp or weft yarns per 1 cm of width, or length of the woven textile.

The term "wale density" means the number of wales of the knitted textile, per 1 dm width of the knitted textile.

The term "nano-micellar composition" means the nano-capsule with dimensions 20-100 nm, that is made of acylated hyaluronan formed into the micellar structure and that carries the active agent in the core.

### Description of the drawings

**Fig. 1A** Shows the centric arrangement of the core and the sheath in the fiber. The core is illustratively pulled out of the sheath.
**Fig. 1B** Shows the core-sheath fiber with the content of magnetic nanoparticles, prepared according to Example 6.
**Fig. 2A** Shows the braided textile of 16 core-sheath fibers of the invention with inserted filling fibers before wetting in water. The fiber is prepared according to method of preparation stated in Example 6 and processed with braiding according to Example 18a.
**Fig. 2B** Shows the braided textile identical with the textile from Fig. 2A after wetting in water, release of hyaluronan and active agent from the core and air-drying.

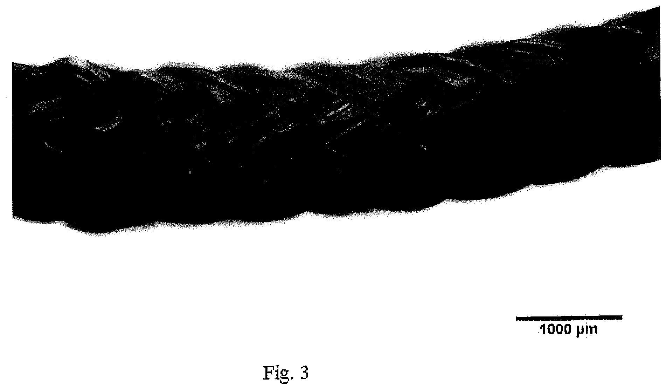

**Fig. 3** Structure of the braided tube of Example 17a.

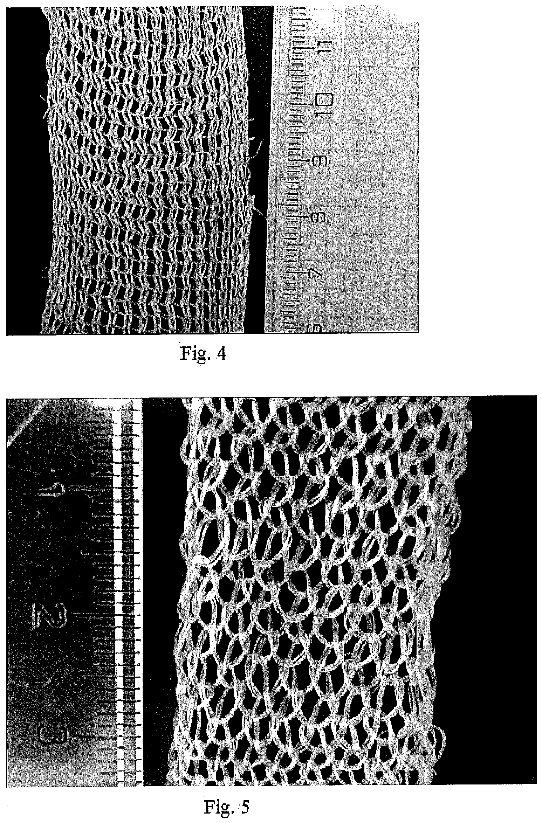

**Fig. 4** Core-sheath fibers processed into the form of the weft knitted textile of Example 22.
**Fig. 5** Core-sheath fibers processed into the form of the warp knitted textile of Example 23.

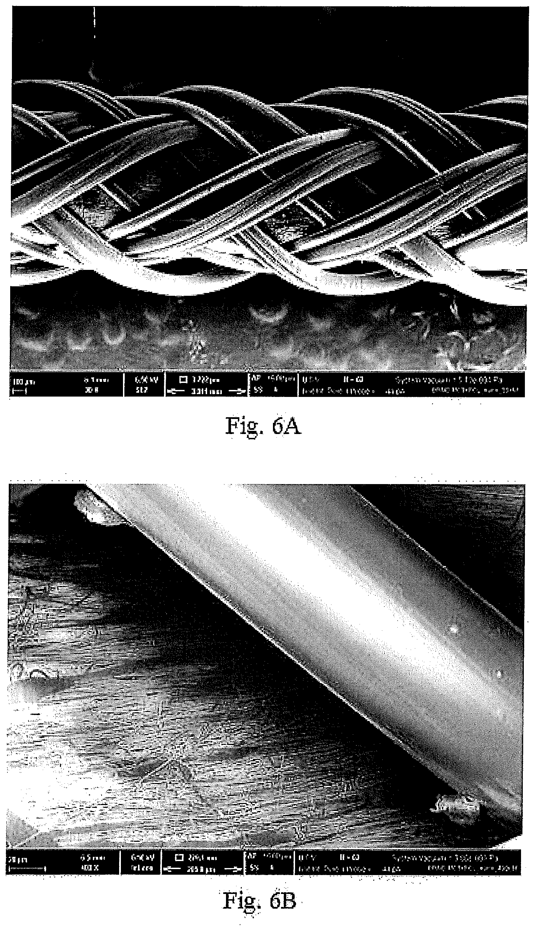

**Fig 6A** The braided thread with filling fibers of Example 18c.
**Fig. 6B** The braided thread with filling fibers of Example 18c - the detail showing filling fibers.

### Examples

Molar mass of hyaluronan was determined using HPLC Shimadzu, with attached light scattering detector midiDAWN Watt Technologies, (so called method SEC-MALLS). If not stated otherwise, stated molecular masses are medium masses.

Shots of core-sheath fibers were taken on the scanning electron microscope:
1) Tescan VEGA II LSU with the wolfram cathode and maximal resolution 3 nm. The accelerating voltage of the primary beam was 5 kV, working distance of 3-4 mm images were taken under high vacuum.
2) ZEISS ULTRA PLUS with Schottky cathode. Microscope reaches maximal resolution 0.8 nm at 30 kV for STEM detector. The accelerating voltage of the primary beam was 5 kV, working distance of 4.6 mm, current value was 40 pA, images were taken with InLens SE detector.

### Example 1

### Preparation of core-sheath fibers from sodium hyaluronate and palmitoyl hyaluronan

For the preparation of the fiber core, it was 0.49 g of sodium hyaluronate (MW 1.57x10⁶ g/mol) dissolved in 32.5 mL of demineralized water. Solution of the sheath was made by mixing 1.65 g of palmitoyl hyaluronan (Mw 2.4x10⁵ g/mol, degree of substitution 28 %) with 15 mL of demineralized water and 15 mL of propan-2-ol. Resulting solutions were degassed and co-extruded using the pair of dosage systems through the two-way coaxial spinneret into the coagulation bath. Extrusion rate was 200 µL.min⁻¹ for both components. The coagulation bath was made of a mixture of 80% D,L-lactic acid and 100% propan-2-ol in volume ratio of 1:4. The resulting core-sheath fiber went through a 0.4 m long bath with the rate of 0.6 m.min⁻¹ and it was continually pulled out by the winding rollers. The fiber was then scoured with 100% propan-2-ol.

The core and the sheath were in volume ratio of 1:3.5. The fiber fineness was 19 tex, strength 1.46 N and its breaking elongation 12.3%.

### Example 2

### Preparation of core-sheath fibers from palmitoyl hyaluronan and sodium hyaluronate

For the preparation of the fiber core, it was 1.5 g of palmitoyl hyaluronate (Mw 2.4x10⁵ g/mol, degree of substitution 28 %) dissolved in 11 mL of demineralized water and 11 mL of propan-2-ol. Solution for the sheath was prepared by mixing 0.66 g sodium hyaluronate (Mw 1.57x10⁶ g/mol) with 22 mL of demineralized water. Resulting solutions were degassed and co-extruded using the pair of dosage systems through the two-way coaxial spinneret into the coagulating bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 1.0 m.min⁻¹ and it was continually pulled out by the winding roller. The fiber was then scoured with 100% propan-2-ol.

The core and the sheath were in volume ratio 2.3:1. The fiber fineness was 22 tex.

### Example 3

### Preparation of core-sheath fibers from sodium hyaluronate and stearoyl hyaluronan

For the preparation of the fiber core, it was 0.49 g of sodium hyaluronate (Mw 1.57x10⁶ g/mol) dissolved in 32.5 mL of demineralized water. The solution of the sheath was made by mixing 0.95 g of stearoyl hyaluronan (Mw 1.3x10⁶ g/mol, degree of substitution 35 %) with 15 mL of demineralized water and 15 mL of propan-2-ol. Resulting solutions were degassed and co-extruded using the pair of dosage systems through the two-way coaxial spinneret into coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.6 m.min⁻¹ and it was continually pulled out by the winding roller. The fiber was then scoured with 100% propan-2-ol.

The core and the sheath were in volume ratio 1:2.1. The fiber fineness was 15 tex.

### Example 4

### Preparation of core-sheath fibers from sodium hyaluronate and palmitoyl hyaluronan with addition of octenidine dihydrochloride into the fiber core

For the preparation of the fiber core, it was 0.375 g of sodium hyaluronate (Mw 1.57x10⁶ g/mol) dissolved in 21.6 mL of demineralized water. 10 mg of octenidine dihydrochloride was added to this solution. The solution of the sheath was made by mixing 0.88 g of palmitoyl hyaluronan (Mw 2.4x10⁵ g/mol, degree of substitution 27 %) with 11 mL of demineralized water and 11 mL of propan-2-ol. Resulting solutions were degassed and co-extruded using the pair of dosage systems through the two-way coaxial spinneret into coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 1.0 m.min⁻¹ and it was continually pulled out by the winding roller. The fiber was then scoured with 100% propan-2-ol.

The core and the sheath were in the volume ratio 1:2.3. The fiber fineness was 20 tex.

### Example 5

### Preparation of core-sheath fibers from sodium hyaluronate and palmitoyl hyaluronan with addition of dexamethasonsodium phosphate

For the preparation of the fiber core, it was 0.3 g of sodium hyaluronate (Mw 1.57x10⁶ g/mol) dissolved in 18 mL of demineralized water. 15 mg of dexamethasonsodium phosphate was added to this solution. The solution for sheath was made by mixing 1.32 g of palmitoyl hyaluronan (Mw 2.4x10⁵ g/mol, degree of substitution 28 %) with 11 mL of demineralized water and 11 mL of propan-2-ol. Resulting solutions were degassed and co-extruded using the pair of dosage systems through the two-way coaxial spinneret into coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.6 m.min⁻¹ and it was continually pulled out by the winding roller. The fiber was then scoured with 100% propan-2-ol.

The core and the sheath were in volume ratio 1:3.6. The fiber fineness was 28 tex.

### Example 6

### Preparation of core-sheath fibers from sodium hyaluronate and palmitoyl hyaluronan with addition of magnetic nanoparticles

For the preparation of the fiber core, it was 0.48 g of sodium hyaluronate (Mw 1.57x10⁶ g/mol) dissolved in 32 mL of demineralized water. 7 mg of dispersed magnetic nano-particles based on iron oxides in ethanol at density of 8.55 g.cm⁻³ (nano-particle size 6 to 10 nm, covered by triethylen glycol) was added to this solution. Solution for the sheath was made by mixing 2.33 g of palmitoyl hyaluronan (Mw 2.70x10⁵ g/mol, degree of substitution 37 %) with 16 mL of demineralized water and 16 mL of propan-2-ol. Resulting solutions were degassed and co-extruded using the pair of dosage systems through the two-way coaxial spinneret into coagulation bath. Extrusion rate was 200 µL.min⁻¹ for both components. The coagulation bath was made of the mixture of 80% D,L-lactic acid and 100% propan-2-ol in volume ratio 1:4. Resulting core-sheath fiber went through 0.4 m long bath with the rate 0.6 m.min⁻¹ and was continually pulled out by the winding roller. The fiber was then scoured with 100% propan-2-ol.

The core and the sheath were in volume ratio 1:5. The fiber fineness was 32 tex, strength 2.62 N and its breaking elongation 8.1%.

### Example 7

### Preparation of core-sheath fibers from sodium hyaluronate with low molar mass and palmitoyl hyaluronan with high molar mass

For the preparation of the fiber core it was 1 g of sodium hyaluronate (Mw 3x10⁵ g/mol) dissolved in 24 mL of demineralized water. Solution for the sheath was made by mixing 1.87 g of palmitoyl hyaluronan (Mw 6.40x10⁵ g/mol, degree of substitution 44 %) with 17 mL of demineralized water and 17 mL of popan-2-ol. Resulting solutions were degassed and co-extruded using the pair of dosage systems through the two-way coaxial spinneret into coagulation bath. Extrusion rate was 250 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 1.0 m.min⁻¹ and was continually pulled out by the winding roller. The fiber was then scoured with 100% propan-2-ol and acetone.

The core and the sheath were in volume ratio 1:1.3. The fiber fineness was 28 tex, strength 2.35 N and its breaking elongation 21.1 %.

### Example 8

### Preparation of the core-sheath fiber of low value of fineness

For the preparation of the fiber core, it was 0.38 g of sodium hyaluronate (Mw 1.66x10⁶ g/mol) dissolved in 25 mL of demineralized water. Solution for the sheath was made by mixing 1.6 g of palmitoyl hyaluronan (Mw 2.80 x10⁵ g/mol, degree of substitution 36 %) with 17 mL of demineralized water and 17 mL of propan-2-ol. Resulting solutions were degassed and co-extruded using the pair of dosage systems through the two-way coaxial spinneret into coagulation bath. Extrusion rate was 200 µL.min⁻¹ for both components. The coagulation bath was made of a mixture of 80% D,L-lactic acid and 100% propan-2-ol in volume ratio 1:4. The resulting core-sheath fiber went through 0.4 m long bath with the rate 1.3 m.min⁻¹ and it was continually pulled out by the winding roller. The fiber was then scoured with 100% propan-2-ol and acetone.

The core and the sheath were in volume ratio 1:3. The fiber fineness was 11 tex, strength 1.41 N and its breaking elongation 15.5 %.

### Example 9

### Preparation of the core-sheath fiber from sodium hyaluronate and palmitoyl hyaluronan with addition of octenidin dihydrochloride in the fiber sheath

For the preparation of the fiber core, it was 0.45 g of sodium hyaluronate (Mw 6.80x10⁵ g/mol) dissolved in 25 mL of demineralized water. Solution for the sheath was made by mixing 1.59 g of palmitoyl hyaluronan (Mw 2.40x10⁵ g/mol, degree of substitution 55 %) with 17 mL of demineralized water and 17 mL of propan-2-ol. 30 mg of octenidine dihydrochloride was added to this solution. Resulting solutions were degassed and co-extruded using the pair of dosage systems through the two-way coaxial spinneret into the coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.8 m.min⁻¹ and it was continually pulled out by the winding roller. The fiber was then scoured with 100% propan-2-ol and acetone.

The core and the sheath were in volume ratio 1:2.5. The fiber fineness was 20 tex, strength 1.97 N and its breaking elongation 15.4 %.

### Example 10

### Preparation of the core-sheath fiber from sodium hyaluronate and palmitoyl hyaluronan with addition of naproxen into the fiber core and octenidine dihydrochloride into the fiber sheath

For the preparation of the fiber core, it was 1.05 g of sodium hyaluronate (Mw 3x10⁵ g/mol) dissolved in 20 mL of demineralized water. 40 mg of sodium hyaluronate derivative with covalently bound naproxen (17% naproxen) was added to this solution. Solution for the fiber sheath was made by mixing 1.59 g palmitoyl hyaluronan (Mw 2.40x10⁵ g/mol, degree of substitution 55 %) with 17 mL of demineralized water and 17 mL of propan-2-ol. 30 mg of octenidine dihydrochloride was added to this solution. Resulting solutions were degassed and co-extruded using the pair of dosage systems through the two-way coaxial spinneret into the coagulation bath. Extrusion rate was 250 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 1.0 m.min⁻¹ and was continually pulled out by the winding roller. The fiber was then scoured with 100% propan-2-ol and acetone.

The core and the sheath were in volume ratio 1.2:1. The fiber fineness was 26 tex, strength 1.58 N and its breaking elongation 19.2 %.

### Example 11

### Preparation of core-sheath fibers from sodium hyaluronate and palmitoyl hyaluronan, wherein different volume ratio of demineralized water and propan-2-ol or another lower alcohol is used in spinning solution of palmitoyl hyaluronan

For the preparation of the fiber core, it was 0.45 g of sodium hyaluronate (Mw 6.80x10⁵ g/mol) dissolved in 25 mL demineralized water. Solution for the fiber sheath was made by mixing
a) 1.60 g of palmitoyl hyaluronan (Mw 2.12x10⁵ g/mol, degree of substitution 58 %) with 17 mL of demineralized water and 17 mL of propan-2-ol (1:1),
b) 1.84 g of palmitoyl hyaluronan (Mw 2.12x10⁵ g/mol, degree of substitution 58 %) with 16 mL of demineralized water and 24 mL of propan-2-ol (4:6)
c) 1.80 g of palmitoyl hyaluronan (Mw 2.12x10⁵ g/mol, degree of substitution 58 %) with 31.5 mL of demineralized water and 3.5 mL of propan-2-ol (9:1)
d) 1.60 g of palmitoyl hyaluronan (Mw 2.12x10⁵ g/mol, degree of substitution 58 %) with 17 mL of demineralized water and 17 mL of ethanol (1:1)
Resulting solutions were degassed and co-extruded using the pair of dosage systems through the two-way coaxial spinneret into the coagulation bath. Extrusion rate was 200 (µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.8 m.min⁻¹ and was continually pulled out by the winding roller. The fiber was then scoured with 100% propan-2-ol and acetone.
a) The core and the sheath were in volume ratio 1:2.5. The fiber fineness was 17 tex, strength 1.99 N and its breaking elongation 18.2 %.
b) The core and the sheath were in volume ratio 1:2.5. The fiber fineness was 17 tex, strength 1.74 N and its breaking elongation 17.8 %.
c) The core and the sheath were in volume ratio 1:2.8. The fiber fineness was 17 tex.
d) The core and the sheath were in volume ratio 1:2.6. The fiber fineness was 15 tex, strength 1.67 N and its breaking elongation 12.6 %.

### Example 12

### Preparation of core-sheath fibers from sodium hyaluronate and palmitoyl hyaluronan in coagulation baths with different ratios of lactic acid, propan-2-ol and water

For the preparation of the fiber core, it was 0.36 g of sodium hyaluronate (Mw 1.66x10⁶ g/mol) dissolved in 25 mL of demineralized water. Solution for the fiber sheath was made by mixing 1.60 g of palmitoyl hyaluronan (Mw 2.80x10⁵ g/mol, degree of substitution 36 %) with 17 mL of demineralized water and 17 ml of propan-2-ol. Resulting solutions were degassed and co-extruded using the pair of dosage systems through the two-way coaxial spinneret into coagulation bath. Extrusion rate was 200 µL.min⁻¹ for both components.
The coagulation bath was made by:
a) The mixture of 80 % D,L-lactic acid and 100 % propan-2-ol in volume ratio 1:4
b) The mixture of 80% D,L-lactic acid and 100% propan-2-ol and demineralized water in volume ratio 2:7:1
c) The mixture of 80% D,L-lactic acid and 100% propan-2-ol and demineralized water in volume ratio 0.5:6:3.5
The resulting core-sheath fiber went through 0.4 m long bath with the rate 1 m.min⁻¹ and was continually pulled out by the winding roller. The fiber was then scoured with 100% propan-2-ol and acetone.
a) The core and the sheath were in volume ratio 1:3. The fiber fineness was 16 tex, strength 2.13 N and its breaking elongation 12.8 %.
b) The core and the sheath were in volume ratio 1:3. The fiber fineness was 16 tex, strength 1.58 N and itsbreaking elongation 14.2 %.
c) The core and the sheath were in volume ratio 1:3. The fiber fineness was 16 tex.

### Example 13

### Preparation of core-sheath fibers from sodium hyaluronate and oleoyl hyaluronan

For the preparation of the fiber core was 0.38 g of sodium hyaluronate (Mw 1.66x10⁶ g/mol) dissolved in 25 mL of demineralized water. Solution for the sheath was made by mixing 1.43 g of oleoyl hyaluronan (Mw 2.8x10⁵ g/mol, degree of substitution 28 %) with 17 mL of demineralized water and 17 mL of propan-2-ol. Resulting solutions were degassed and co-extruded using the pair of dosage systems through the two-way coaxial spinneret into the coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.9 m.min⁻¹ and it was continually pulled out by the winding roller. The fiber was then scoured with 100% propan-2-ol.

The core and the sheath were in volume ratio 1:2.8. The fiber fineness was 13 tex, strength 1.35 N and its breaking elongation 11.2 %.

### Example 14

### Preparation of core-sheath fibers from oleoyl hyaluronan and palmitoyl hyaluronan

### a) Degree of substitution in the core 28 %, degree of substitution in the sheath 36 %

For the preparation of the fiber core, it was 0.77 g of oleoyl hyaluronan (Mw 2.8x10⁵ g/mol, degree of substitution 28 %) dissolved in 14 mL of demineralized water. Solution for the sheath was made by mixing 1.77 g of palmitoyl hyaluronan (Mw 2.8x10⁵ g/mol, degree of substitution 36 %) with 17 mL of demineralized water and 17 mL of propan-2-ol. Resulting solutions were degassed and co-extruded using the pair of dosage systems through the two-way coaxial spinneret into the coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.9 m.min⁻¹ and was continually pulled out by the winding roller. The fiber was then scoured with 100% ethanol.

The core and the sheath were in volume ratio 1:2. The fiber fineness was 20 tex, strength 1.31 N and its breaking elongation 16.9 %.

### b) Degree of substitution in the core 24 %, degree of substitution in the sheath 50 %

For the preparation of the fiber core, it was 0.77 g of oleoyl hyaluronan (Mw 2.16x10⁵ g/mol, degree of substitution 24 %) dissolved in 12 mL of demineralized water and 12 mL of propan-2-ol. Solution for the sheath was made by mixing 1.41 g of palmitoyl hyaluronan (Mw 2.04x10⁵ g/mol, degree of substitution 50 %) with 15 mL of demineralized water and 15 mL of propan-2-ol. Resulting solutions were degassed and co-extruded using the pair of dosage systems through the two-way coaxial spinneret into the coagulation bath. Extrusion rate was 200 µL.min⁻¹ for both components. The coagulation bath was made of a mixture of 80% D,L-lactic acid and 100% propan-2-ol in volume ratio 1:4. The resulting core-sheath fiber went through 0.4 m long bath with the rate 0.9 m.min⁻¹ and it was continually pulled out by the winding roller. The fiber was then scoured with 100% ethanol and additional 4 hours in acetone.

The core and the sheath were in volume ratio 1:1.5. The fiber fineness was 20 tex, strength 2.03 N and its breaking elongation 22.1 %.

### Example 15

### Preparation of core-sheath fibers from palmitoyl hyaluronan of different degree of substitution in the fiber core and sheath

### a) Degree of substitution in the core 16 %, degree of substitution in the sheath 75 %

For the preparation of the fiber core, it was 0.77 g of palmitoyl hyaluronan (Mw 2.16x10⁵ g/mol, degree of substitution 16 %) dissolved in 14 mL of demineralized water and 14 mL of propan-2-ol. Solution for the sheath was made by mixing 1.87 g of palmitoyl hyaluronan (Mw 2.8x10⁵ g/mol, degree of substitution 75 %) with 17 mL of demineralized water and 17 mL of propan-2-ol. Resulting solutions were degassed and co-extruded using the pair of dosage systems through the two-way coaxial spinneret into the coagulation bath. Extrusion rate was 200 µl.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.9 m.min⁻¹ and it was continually pulled out by the winding roller. The fiber was then scoured with 100% ethanol.

The core and the sheath were in volume ratio 1:2. The fiber fineness was 20 tex, strength 1.11 N and its breaking elongation 9.5 %.

### b) Degree of substitution in core 12 %, degree of substitution in sheath 60 %

For the preparation of the fiber core was 0.77 g of palmitoyl hyaluronan (Mw 3.2x10⁵ g/mol, degree of substitution 12 %) dissolved in 12 mL of demineralized water and 12 mL of propan-2-ol. Solution for the sheath was made by mixing 1.41 g of palmitoyl hyaluronan (Mw 2.03x10⁵ g/mol, degree of substitution 60 %) with 15 mL of demineralized water and 15 mL of propan-2-ol. Resulting solutions were degassed and co-extruded using the pair of dosage systems through the two-way coaxial spinneret into coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.9 m.min⁻¹ and it was continually pulled out by the winding roller. The fiber was then scoured with 100% ethanol and additional 4 hours in acetone.

The core and the sheath were in volume ratio 1:1.5. The fiber fineness was 20 tex, strength 1.96 N and its breaking elongation 23.0 %.

### c) Degree of substitution in the core 5 %, degree of substitution in the sheath 44 %

For the preparation of the fiber core, it was 0.77 g of palmitoyl hyaluronan (Mw 3.2x10⁵ g/mol, degree of substitution 5 %) dissolved in 12 mL of demineralized water and 12 mL of propan-2-ol. Solution for sheath was made by mixing 1.41 g of palmitoyl hyaluronan (Mw 2.04x10⁵ g/mol, degree of substitution 44 %) with 15 mL of demineralized water and 15 mL of propan-2-ol. Resulting solutions were degassed and co-extruded using the pair of dosage systems through the two-way coaxial spinneret into coagulation bath. Extrusion rate was 200 µL.min⁻¹ for both components. Coagulation bath was made of a mixture of 80% D,L-lactic acid and 100% propan-2-ol in volume ratio 1:4. The resulting core-sheath fiber went through 0.4 m long bath with the rate 0.9 m.min⁻¹ and it was continually pulled out by the winding roller. The fiber was then scoured with 100% ethanol and additional 4 hours in acetone.

The core and the sheath were in volume ratio 1:1.5. The fiber fineness was 24 tex, strength 2.45 N and its breaking elongation 24.3 %.

### Example 16

### Preparation of core-sheath fibers from sodium hyaluronate and oleoyl hyaluronan with addition of micelles containing fluorescent agent into the fiber core

For the preparation of the fiber core was 0.38 g of sodium hyaluronate (Mw 1.66x10⁶ g/mol) dissolved in 25 mL of demineralized water. 7.6 mg of aqueous solution of the nano-micellar composite on the basis of polysaccharide containing dye Nile red (solution concentration 2 wt%) was added to this solution. Solution for the sheath was made by mixing 1.43 g of oleoyl hyaluronan (Mw 2.8x10⁵ g/mol, degree of substitution 28 %) with 17 mL of demineralized water and 17 mL of propan-2-ol. Resulting solutions were degassed and co-extruded using the pair of dosage systems through the two-way coaxial spinneret into the coagulation bath. Extrusion rate was 200 µL.min⁻¹ for both components. The coagulation bath was made of the mixture of 80% D,L-lactic acid and 100% propan-2-ol in volume ratio 1:4. The resulting core-sheath fiber went through 0.4 m long bath with the rate 0.9 m.min⁻¹ and it continually pulled out by the winding roller. The fiber was then scoured with 100% propan-2-ol and acetone.

The core and the sheath were in volume ratio 1:2.8. The fiber fineness was 14 tex, strength 1.49 N and its breaking elongation 16.6 %.

### Example 17

### Preparation of the braided textile from core-sheath fibers based on hyaluronan

### a) Preparation of the braided tube from core-sheath fibers based on hyaluronan

Yarn made of the single core-sheath fiber (monofilament) from palmitoyl hyaluronan and sodium hyaluronate with content of magnetic nanoparticles prepared according to Example 6 was rewound to 16 bobbins using the winding machine and then processed using STEEGER horizontal braiding machine with the braid body set with 16 bobbins. The winding speed was 30 m/min, braiding head speed was 50 rpm. The resulting braided tube from 16 monofilament/yarns in twill showed braiding density 13 cm⁻¹, braiding angle 30° and diameter 1.25 mm.

### b) Preparation of the braided thread from mixed yarn

First was produced yarn from the bundle of two fibers (monofilaments): the core-sheath fiber from palmitoyl hyaluronan and sodium hyaluronate with content of magnetic nano-particles made according to Example 6 and polypropylene fiber of diameter 0.08 mm and fineness 5.2 tex by Wetelen. Yarn was made by grouping of these monofilaments during rewinding on a STEEGER winding machine. The winding speed was 27 m/min and tension 8cN. 8 bobbins with upwound blended yarn was gradually prepared. The yarn fineness was 37 tex. Yarn was then processed using a STEEGER horizontal braiding machine with the braid body set with 8 bobbins. The braiding head speed was 30 rpm. The resulting braided thread made of 8 yarns showed braiding density 10 cm⁻¹, braiding angle 20°and diameter 0.9 mm.

### Example 18

### Preparation of the braided tube from core-sheath fibers based on hyaluronan containing filling fibers

### a) Filling fibers from PLLA of diameter 0.076 mm

In manufacturing of the braided tube according to the method from Example 17a, the bundle of 30 filling fibers of polylactic acid (PLLA) of diameter 0.076 mm by Luxilon was inserted into the hollow. The resulting braided tube showed braiding density 13 cm⁻¹, braiding angle 30° and diameter 1.25 mm. The resulting tube was submerged in water, wherein the fibers swelled during several minutes, the sheath thereof started to rupture and after 10 minutes core with active agent release was apparent. After 4 hours was the core completely spilled out.

### b) Filling fibers from polyester of diameter 0.018 mm

In manufacturing of braided tube according to method from Example 17b, the multifilament made of 200 fibers of polyester of diameter 0.018 mm was inserted in the hollow. The resulting braided tube showed braiding density 10 cm⁻¹, braiding angle 28° and diameter 0.95 mm.

### c) Filling fibers from polycaprolactone of diameter 0.000 54 mm (nano-fibers)

In manufacturing of braided tube according to method from Example 17b, the polyester multifilament with its surface covered with nano-fiber layer of polycaprolactone (Mw 8.0x10⁴ g/mol; nano-fiber layer was made using method of electrostatic spinning on apparatus 4SPIN) was inserted into the hollow. Nano-fiber diameter was 0.000 54 mm. The resulting braided tube showed braiding density 10 cm⁻¹, braiding angle 31° and diameter 1.5 mm.

### Example 19

### Preparation of the yarn of core-sheath fibers based on hyaluronan

### a) Simple yarn of fineness 60 tex

3 monofilaments of core-sheath fibers from sodium hyaluronate and palmitoyl hyaluronan (fibers made according to Example 1) were twisted on a ring-twisting machine at the feeding speed of 8 m/min and spindle rotation speed 1000 min⁻¹. The resulting yarn had fineness 60 tex and twist 125 m⁻¹.

### b) Plied yarn of fineness 300 tex

3 monofilaments of core-sheath fibers from sodium hyaluronate and palmitoyl hyaluronan (fibers prepared according to Example 6) were twisted on the ring-twisting machine at the feeding speed 9 m/min and spindle rotation speed 1000 min⁻¹. This simple yarn had fineness 100 tex and left twist 110 m⁻¹. Then 3 such simple yarns were grouped and twisted on the ring-twisting machine at the feeding speed 12 m/min and spindle rotation speed 1000 min⁻¹. The resulting plied yarn had fineness 300 tex and right twist 85 m⁻¹.

### Example 20

### Preparation of the blended yarn from core-sheath fibers based on hyaluronan and PLLA fibers

1 monofilament of the core-sheath fiber of native hyaluronan and palmitoyl hyaluronan (fibers were made according to Example 12b) and 2 monofilaments of polylactic acid (PLLA) of diameter 0.076 mm were twisted on the ring-twisting machine at the feeding speed 8 m/min and spindle rotation speed 1000 min⁻¹. The resulting yarn had fineness 30 tex and twist 125 m⁻¹.

### Example 21

### Preparation of the braided tube from blended yarn containing core-sheath fibers based on hyaluronan and PLLA fiber

Yarn prepared according to Example 20 was rewound using the winding machine for 8 bobbins and then processed on STEEGER horizontal braiding machine with the braiding body set with 8 bobbins. The winding speed was 30 m/min, braiding body speed was 70 rpm. The resulting braided thread made of 8 yarns in twill showed braiding density of 15 cm⁻¹, braiding angle 30° and diameter 0.8 mm.

### Example 22

### Preparation of the weft-knitted textile containing core-sheath fibers based on hyaluronan

Yarn manufactured according to Example 19 was further textile processed on the semi-production hand driven weft-knitting machine (in gauge of 10 needles/inch - knitted with omitting every second needle) into double-faced knitted textile. The density of resulting knitted textile was 8 wales per centimeter and 4 courses per centimeter.

### Example 23

### Preparation of the warp knitted textile containing core-sheath fibers based on hyaluronan

Yarn made according to Example 19 was further textile processed on the warp knitting machine - raschel machine by COMEZ (in gauge 12 needles/inch) into the form of single-faced knitted textile in the tricot pattern. The density of resulting knitted textile was 4 wales per centimeter and 5 courses per centimeter.

## Claims

1. Endless fiber of the type core-sheath on the basis of hyaluronan or C₁₁-C₁₈ acylated derivative thereof, **characterized in that** it comprises a combination of hyaluronan and acylated derivative thereof or a combination of acylated derivatives thereof, the fiber core and the sheath being in any of the following arrangements:
A) the core contains hyaluronic acid or a salt thereof, the sheath contains C₁₁-C₁₈ acylated hyaluronan;
B) the core contains C₁₁-C₁₈ acylated hyaluronan, the sheath contains hyaluronic acid or a salt thereof;
C) the core contains C₁₁-C₁₈ acylated hyaluronan, the sheath contains different C₁₁-C₁₈ acylated hyaluronan differing in C₁₁-C₁₈ acyl from C₁₁-C₁₈ acylated hyaluronan of the core, the degree of substitution of C₁₁-C₁₈ acylated hyaluronan in the sheath being identical or different from the degree of substitution of C₁₁-C₁₈ acylated hyaluronan in the core; or
D) the core contains C₁₁-C₁₈ acylated hyaluronan, the sheath contains different C₁₁-C₁₈ acylated hyaluronan differing in degree of substitution of C₁₁-C₁₈ acylated hyaluronan of the core.

2. Fiber according to claim 1, **characterized in that** C₁₁-C₁₈ acylated hyaluronan is acylated in primary alcohol of *N*-acetyl-glucosamine.

3. Fiber according to claim 1 or claim 2, **characterized in that** molar mass of C₁₁-C₁₈ acylated hyaluronan is in the range 1 x 10⁵ to 7 x 10⁵ g/mol, preferably 2 x 10⁵ to 3 x 10⁵ g/mol.

4. Fiber according to any one of preceding claims, **characterized in that** the degree of substitution of C₁₁-C₁₈ acylated hyaluronan of arrangement A or B is in the range 5 to 80 %, preferably 30 to 60 %.

5. Fiber according to any one of claims 1 to 3, **characterized in that** the degree of substitution of C₁₁-C₁₈ acylated hyaluronan contained in the core of arrangement C is in the range 5 to 80 %, preferably 5 to 29 %, more preferably 10 to 20 % and the degree of substitution of C₁₁-C₁₈ acylated hyaluronan contained in the sheath of arrangement C is in the range 5 to 80 %, preferably in the range 30 to 80 %, more preferably 40 to 60 %; or the degree of substitution of C₁₁-C₁₈ acylated hyaluronan contained in the core of arrangement D is in the range 5 to 29 %, preferably 10 to 20 % and the degree of substitution of C₁₁-C₁₈ acylated hyaluronan contained in the sheath of arrangement D is in the range 30 to 80 %, preferably in the range 40 to 60 %.

6. Fiber according to any one of preceding claims, **characterized in that** C₁₁-C₁₈ acylated hyaluronan is selected from a group containing palmitoyl hyaluronan, stearoyl hyaluronan or oleoyl hyaluronan.

7. Fiber according to any one of claims 1 to 4 or according to claim 6, **characterized in that** hyaluronic acid and/or a salt thereof has molar mass in the range 1 x 10⁵ to 2 x 10⁶ g/mol, preferably 8 x 10⁵ to 1.8 x 10⁶ g/mol, the salt of hyaluronic acid being selected from a group containing alkali metal ions, alkaline earth metals ions, preferably Na⁺, K⁺, Ca²⁺ or Mg²⁺.

8. Fiber according to any one of preceding claims, **characterized in that** fiber fineness is in the range 10 to 40 tex, preferably 15 to 35 tex, more preferably 22 to 28 tex.

9. Fiber according to any one of preceding claims, **characterized in that** the volume ratio core:sheath is in the range 3:1 to 1:6, preferably 1:3 to 1:5, more preferably 1:4.

10. Fiber according to any one of preceding claims, **characterized in that** it contains at least one active agent.

11. Fiber according to claim 10, **characterized in that** both core and sheath contain at least one identical or different active agent.

12. Fiber according to claim 10 or claim 11, **characterized in that** the active agent is selected from a group containing antibacterial, antiseptic, antibiotic, anti-inflammatory, hemostatic agents, anesthetics, cytostatics, hormones, immunomodulators, immunosuppressives or agents for contrast imagining, preferably magnetic nanoparticles.

13. Fiber defined in any one of claims 1 to 12 for use in manufacturing of yarns, braided, woven, knitted or non-woven textiles.

14. Method of preparation of fibers defined in any one of claims 1 to 13, **characterized in that** either for the preparation of the fiber of arrangement A or B the first spinning solution containing hyaluronic acid or a salt thereof of concentration in the range 1 to 8 wt% is separately prepared in water and the second spinning solution containing C₁₁-C₁₈ acylated hyaluronan of concentration 2 to 8 wt% is prepared in the mixture of water and lower alcohol, wherein water content is in the range 30 v/v% to 90 v/v%, lower alcohol content is in the range 10 v/v% to 70 v/v%, or for the preparation of the fiber of arrangement C or D the first spinning solution containing C₁₁-C₁₈ acylated hyaluronan of concentration in the range 2 to 8 wt% is separately prepared in a mixture of water and lower alcohol and the second spinning solution containing different C₁₁-C₁₈ acylated hyaluronan of concentration 2 to 8 wt% is prepared in a mixture of water and lower alcohol, wherein water content in both solutions is in the range 30 v/v% to 90 v/v%, lower alcohol content is in the range 10 v/v% to 70 v/v%,
after which both the first and second spinning solution are extruded together into the coagulation bath containing 2 to 40 wt% of organic acid, preferably lactic acid, at least 50 wt% of lower alcohol, preferably is used ethanol, propan-1-ol or propan-2-ol, and 2 to 48 wt% of water resulting in fiber of arrangement A or B or C or D, that is consequently scoured with lower alcohol and dried.

15. Method according to claim 14, **characterized in that** the first spinning solution and/or the second spinning solution contain an active agent.

16. Method according to claim 14, **characterized in that** the first spinning solution and/or the second spinning solution contain nanomicellar composition based on acylated hyaluronan containing the active agent.

17. Staple fibers made from fibers defined in any one of claims 1 to 13.

18. Non-woven textile made of staple fibers defined in claim 17.

19. Yarn made of at least one fiber defined in any one of claims 1 to 13.

20. Yarn according to claim 19, **characterized in that** it is formed of a bundle of fibers containing 2 to 10 fibers, preferably 3 to 6.

21. Yarn according to claim 20, **characterized in that** it is in the form of twisted bundle of fibers.

22. Yarn formed of at least two fibers, **characterized in that** at least one fiber is fiber defined in any one of claims 1 to 13, and at least one fiber is fiber selected from a group of fibers of other biodegradable materials, the biodegradable material being selected from a group comprising polylactic acid, polyglycolic acid, copolymer of polylactic and polyglycolic acid, polycaprolactone, polydioxanone or polyhydroxyalkanoates, preferably of polylactic acid or copolymer of polylactic acid and polyglycolic acid.

23. Yarn according to any one of claims 19 to 22, **characterized in that** it has fineness in the range 10 to 400 tex, preferably 30 to 100 tex.

24. Braided textile, woven textile or knitted textile, **characterized in that** it contains at least one yarn defined in claims 19 to 23.

25. Braided, woven or knitted textile according to claim 24, **characterized in that** it is made of yarn defined in any one of claims 19 to 23.

26. Braided, woven or knitted textile according to claim 24 or claim 25, **characterized in that** fiber core and sheath in the yarn are in arrangement A, C or D, whereas in the case of arrangement A there is the degree of substitution C₁₁-C₁₈ acylated hyaluronan in the range 30 to 80 % in the sheath, preferably 40 to 60 % and in the case of arrangement C or D there is the degree of substitution of C₁₁-C₁₈ acylated hyaluronan in the core in the range 5 to 29 %, preferably 10 to 20 % and the degree of substitution of C₁₁-C₁₈ acylated hyaluronan is in the range 30 to 80 %, preferably 40 to 60 % in the sheath.

27. Braided, woven or knitted textile according to any one of claims 24 to 26, **characterized in that** it is in the form of linear, flat or tubular textile.

28. Braided textile according to any one of claims 24 to 27, **characterized in that** it contains at least 8 yarns, containing at least one filling fiber inside the textile.

29. Braided textile according to claim 28, **characterized in that** the diameter of filling fiber is in the range 0.0001 to 1 mm, preferably 0.01 to 0.1 mm.

30. Braided textile according to claim 28 or claim 29, **characterized in that** the filling fiber is formed of a polymer selected from a group containing hyaluronic acid or derivatives thereof, polylactic acid, polyglycolic acid, copolymer of polylactic and polyglycolic acid, polycaprolactone, polydioxanone, polyhydroxyalkanoates, polyethylene, polypropylene, polyetherester, polyamide or polyester, preferably polylactic acid or copolymer of polylactic acid and polyglycolic acid.

31. Braided textile according to any one of claims 24 to 30, **characterized in that** the braiding angle is at least 20°, preferably at least 25°, whereas it applies that if
the yarn fineness is x tex, than the braiding density of the yarn is at least y cm⁻¹, preferably z cm⁻¹, as is stated in table below
| x | y | z |
|---|---|---|
| Less than 20 | 15 | 20 |
| 20 to 30 | 12 | 15 |
| 30 to 50 | 9 | 12 |
| 50 to 80 | 7 | 9 |
| 80 to 120 | 6 | 7 |
| 120 to 180 | 5 | 6 |
| 180 to 270 | 4 | 5 |
| More than 270 | 3 | 4 |

32. Woven textile according to any one of claims 24 to 27, **characterized in that** if the yarn fineness is a tex, then fabric sett is at least b cm⁻¹, preferably at least c cm⁻¹, as is stated in table below
| a | b | c |
|---|---|---|
| Less than 20 | 25 | 35 |
| 20 to 30 | 18 | 25 |
| 30 to 50 | 14 | 20 |
| 50 to 80 | 11 | 16 |
| 80 to 120 | 9 | 13 |
| 120 to 180 | 7 | 11 |
| 180 to 270 | 6 | 9 |
| More than 270 | 5 | 7 |

33. Knitted textile according to any one of claims 24 to 27, **characterized in that** if the yarn fineness is e tex, then wale density is at least f dm⁻¹, preferably at least g dm⁻¹, as is stated in table below
| e | f | g |
|---|---|---|
| Less than 20 | 20 | 27 |
| 20 to 30 | 18 | 23 |
| 30 to 50 | 16 | 20 |
| 50 to 100 | 14 | 18 |
| More than 270 | 12 | 16 |

34. Use of braided, woven or knitted textile according to any one of claims 24 to 33 and non-woven textile according to claim 18 in the area of medicine, tissue engineering and systems for controlled release.

## Patentansprüche

1. Endlose Faser mit einem Kern-Mantel-Aufbau auf der Basis von Hyaluronat oder eines C₁₁-C₁₈-acylierten Derivats desselben, **dadurch gekennzeichnet, dass** die Faser eine Kombination von Hyaluronat und einem acylierten Derivat desselben oder eine Kombination von acylierten Derivaten desselben umfasst, wobei der Kern und der Mantel der Faser in einer der nachfolgenden Anordnungen vorliegen können:
A) der Kern enthält Hyaluronsäure oder ein Salz derselben, der Mantel enthält C₁₁-C₁₈-acyliertes Hyaluronat;
B) der Kern enthält C₁₁-C₁₈-acyliertes Hyaluronat, der Mantel enthält Hyaluronsäure oder ein Salze derselben;
C) der Kern enthält C₁₁-C₁₈-acyliertes Hyaluronat, der Mantel enthält ein unterschiedliches C₁₁-C₁₈-acyliertes Hyaluronat, das sich durch die C₁₁-C₁₈-Acylgruppe von dem in dem Kern enthaltenen C₁₁-C₁₈-acylierten Hyaluronats unterscheidet, wobei der Substitutionsgrad des in dem Mantel enthaltenen C₁₁-C₁₈-acylierten Hyaluronats identisch mit oder verscheiden von dem Substitutionsgrad des in dem Kern enthaltenen C₁₁-C₁₈-acylierten Hyaluronats ist; oder
D) der Kern enthält C₁₁-C₁₈-acyliertes Hyaluronat, der Mantel enthält ein unterschiedliches C₁₁-C₁₈-acyliertes Hyaluronat, dessen Substitutionsgrad verschieden von dem Substitutionsgrad des in dem Kern enthaltenen C₁₁-C₁₈-acylierten Hyaluronats ist.

2. Faser nach Anspruch 1, **dadurch gekennzeichnet, dass** das C₁₁-C₁₈-acylierte Hyaluronat in einem primären Alkohol von *N*-Acetyl-Glukosamin acyliert ist.

3. Faser nach Anspruch 1 oder Anspruch 2, **dadurch gekennzeichnet, dass** die molare Masse des C₁₁-C₁₈-acylierten Hyaluronats im Bereich von 1 x 10⁵ bis 7 x 10⁵ g/mol, vorzugsweise im Bereich von 2 x 10⁵ bis 3 x 10⁵ g/mol, liegt.

4. Faser nach einem der vorhergehenden Ansprüche, **dadurch gekennzeichnet, dass** der Substitutionsgrad des C₁₁-C₁₈-acylierten Hyaluronats nach der Anordnung A oder B im Bereich von 5 bis 80 %, vorzugsweise im Bereich von 30 bis 60 %, liegt.

5. Faser nach einem der Ansprüche 1 bis 3, **dadurch gekennzeichnet, dass** der Substitutionsgrad des in dem Kern nach der Anordnung C enthaltenen C₁₁-C₁₈-acylierten Hyaluronats im Bereich von 5 bis 80 %, vorzugsweise im Bereich von 5 bis 29 %, vorzüglicherweise im Bereich von 10 bis 20 % liegt und der Substitutionsgrad des in dem Mantel nach der Anordnung C enthaltenen C₁₁-C₁₈-acylierten Hyaluronats im Bereich von 5 bis 80 %, vorzugsweise im Bereich von 30 bis 80 %, vorzüglicherweise im Bereich von 40 bis 60 % liegt, oder dass der Substitutionsgrad des in dem Kern nach der Anordnung D enthaltenen C₁₁-C₁₈-acylierten Hyaluronats im Bereich von 5 bis 29 %, vorzugsweise im Bereich von 10 bis 20 % liegt und der Substitutionsgrad des in dem Mantel nach der Anordnung D enthaltenen C₁₁-C₁₈-acylierten Hyaluronats im Bereich von 30 bis 80 %, vorzugsweise im Bereich von 40 bis 60 % liegt.

6. Faser nach einem der vorhergehenden Ansprüche, **dadurch gekennzeichnet, dass** das C₁₁-C₁₈-acylierte Hyaluronat aus einer Gruppe ausgewählt ist, die Palmitoyl-Hyaluronat, Stearoyl-Hyaluronat oder Oleoyl-Hyaluronat umfasst

7. Faser nach einem der Ansprüche 1 bis 4 oder nach Anspruch 6, **dadurch gekennzeichnet, dass** Hyaluronsäure oder ein Salz derselben eine im Bereich von 1 x 10⁵ bis 2 x 10⁶ g/mol, vorzugsweise im Bereich von 8 x 10⁵ bis 1,8 x 10⁶ g/mol, liegende molare Masse aufweist, wobei das Salz der Hyaluronsäure aus einer Gruppe ausgewählt ist, die Ionen alkalischer Metalle oder Ionen alkalischer Erdmetalle, vorzugsweise Na⁺, K⁺, Ca²⁺ oder Mg²⁺, umfasst.

8. Faser nach einem der vorhergehenden Ansprüche, **dadurch gekennzeichnet, dass** die Feinheit der Faser im Bereich von 10 bis 40 tex, vorzugsweise im Bereich von 15 bis 35 tex, vorzüglicherweise im Bereich von 22 bis 28 tex liegt.

9. Faser nach einem der vorhergehenden Ansprüche, **dadurch gekennzeichnet, dass** das Volumenverhältnis Kern : Mantel im Bereich von 3:1 bis 1:6, vorzugsweise im Bereich von 1:3 bis 1:5, liegt und vorzüglicherweise 1:4 beträgt.

10. Faser nach einem der vorhergehenden Ansprüche, **dadurch gekennzeichnet, dass** sie wenigstens eine wirksame Substanz enthält.

11. Faser nach Anspruch 10, **dadurch gekennzeichnet, dass** sowohl der Kern als auch der Mantels mindestens eine identische oder unterschiedliche wirksame Substanz enthalten.

12. Faser nach Anspruch 10 oder Anspruch 11, **dadurch gekennzeichnet, dass** die wirksame Substanz aus einer Gruppe ausgewählt ist, die antibakterielle, antiseptische, antibiotische, entzündungshemmende oder hämostatische Substanzen, Anästhetika, Zytostatika, Hormone, Immunomodulatore, Immunsuppressiva oder Kontrastsubstanzen für Abbildungsverfahren, vorzugsweise magnetische Nanopartikel, umfasst.

13. Faser nach einem der Ansprüche 1 bis 12 zur Anwendung bei der Herstellung von Garnen sowie geflochtenen, gewebten, gestrickten oder ungewebten Textilien.

14. Verfahren zur Vorbereitung der in einem der Ansprüche 1 bis 13 definierten Fasern, **dadurch gekennzeichnet, dass** entweder bei der Vorbereitung der Fasern in der Anordnung A oder B die erste zu verspinnende Lösung, die Hyaluronsäure oder ein Salz derselben in einer im Bereich von 1 bis 8 Gew.-% liegenden Konzentration enthält, getrennt im Wasser vorbereitet wird und die zweite zu verspinnende Lösung, die das C₁₁-C₁₈-acylierte Hyaluronat in einer im Bereich von 2 bis 8 Gew.-% liegenden Konzentration enthält, in einem Gemisch von Wasser und einem niedrigen Alkohol vorbereitet wird, wobei der Gehalt an Wasser im Bereich von 30 Vol.-% bis 90 Vol.% und der Gehalt an dem niedrigen Alkohol im Bereich von 10 Vol.-% bis 70 Vol.-% liegt, oder bei der Vorbereitung der Fasern in der Anordnung C oder D die erste zu verspinnende Lösung, die ein C₁₁-C₁₈-acyliertes Hyaluronat in einer im Bereich von 2 bis 8 Gew.-% liegenden Konzentration enthält, getrennt in einem Gemisch von Wasser und einem niedrigen Alkohol vorbereitet wird und die zweite zu verspinnende Lösung, die ein unterschiedliches C₁₁-C₁₈-acyliertes Hyaluronat in einer im Bereich von 2 bis 8 Gew.-% liegenden Konzentration enthält, in einem Gemisch von Wasser und einem niedrigen Alkohol vorbereitet wird, wobei in beiden Lösungen der Gehalt an Wasser im Bereich von 30 Vol.-% bis 90 Vol.% und der Gehalt an dem niedrigen Alkohol im Bereich von 10 Vol.-% bis 70 Vol.-% liegt,
worauf sowohl die erste als auch die zweite zu verspinnende Lösung gemeinsam in ein Koagulierbad extrudiert werden, das eine organische Säure, vorzugsweise Milchsäure, mit einem Gewichtsanteil von 2 bis 40 %, einen niedrigen Alkohol mit einem Gewichtsanteil von mindestens 50 %, vorzugsweise Ethanol, Propan-1-ol oder Propan-2-ol, sowie Wasser mit einem Gewichtsanteil von 2 bis 48 % enthält, wodurch eine Faser in einer der Anordnungen A oder B oder C oder D erhalten wird, die abschließend mit einem niedrigen Alkohol durchgewaschen wird und getrocknet wird.

15. Verfahren nach Anspruch 14, **dadurch gekennzeichnet, dass** die erste zu verspinnende Lösung und/oder die zweite zu verspinnende Lösung eine wirksame Substanz enthalten.

16. Verfahren nach Anspruch 14, **dadurch gekennzeichnet, dass** die erste zu verspinnende Lösung und/oder die zweite zu verspinnende Lösung eine nanomizelläre Zusammensetzung auf der Basis eines acylierten Hyaluronats enthalten, die eine wirksame Substanz umfasst.

17. Staple-Fasern, hergestellt aus Fasern nach einem der Ansprüche 1 bis 13.

18. Ungewebte Textilie, hergestellt aus den Staple-Fasern nach Anspruch 17.

19. Garn, hergestellt aus mindestens einer Faser nach einem der Ansprüche 1 bis 13.

20. Garn nach Anspruch 19, **dadurch gekennzeichnet, dass** es aus einem Faserbündel hergestellt ist, das 2 bis 10, vorzugsweise 3 bis 6 Fasern umfasst.

21. Garn nach Anspruch 20, **dadurch gekennzeichnet, dass** es in der Form eines zusammengedrehten Faserbündel vorliegt.

22. Garn, gebildet von mindestens zwei Fasern, **dadurch gekennzeichnet, dass** zumindest eine der Fasern eine in einem der Ansprüche 1 bis 13 definierte Faser ist, und zumindest eine Faser aus einer Gruppe von aus anderen biologisch abbaubaren Werkstoffen hergestellten Fasern ausgewählt ist, wobei der biologisch abbaubare Werkstoff aus einer Gruppe ausgewählt ist, die Polymilchsäure, Polyglykolsäure, ein Kopolymer der Polymilchsäure und Polyglykolsäure, Polycaprolacton, Polydioxanon oder Polyhydroxyalkanoate, vorzugsweise Polymilchsäure oder ein Kopolymer der Polymilchsäure und Polyglykolsäure, umfasst.

23. Garn nach einem der Ansprüche 19 bis 22, **dadurch gekennzeichnet, dass** seine Feinheit im Bereich von 10 bis 400 tex, vorzugsweise im Bereich von 30 bis 100 tex liegt.

24. Geflochtene Textilie, gewebte Textilie oder gestrickte Textilie, **dadurch gekennzeichnet, dass** sie mindestens ein in den Ansprüchen 19 bis 23 definiertes Garn enthält.

25. Geflochtene, gewebte oder gestrickte Textilie nach Anspruch 24, **dadurch gekennzeichnet, dass** sie aus dem in einem der Ansprüchen 19 bis 23 definierten Garn hergestellt ist.

26. Geflochtene, gewebte oder gestrickte Textilie nach Anspruch 24 oder Anspruch 25, **dadurch gekennzeichnet, dass** der Kern und der Mantel der in dem Garn enthaltenen Faser in der Anordnung A, C oder D vorliegen, wobei im Fall der Anordnung A der Substitutionsgrad des C₁₁-C₁₈-acylierten Hyaluronats im Mantel im Bereich von 30 bis 80 %, vorzugsweise im Bereich von 40 bis 60 % liegt und im Fall einer der Anordnungen C oder D der Substitutionsgrad des C₁₁-C₁₈-acylierten Hyaluronats im Kern im Bereich von 5 bis 29 %, vorzugsweise im Bereich von 10 bis 20 % liegt und der Substitutionsgrad des C₁₁-C₁₈-acylierten Hyaluronats im Mantel im Bereich von 30 bis 80 %, vorzugsweise im Bereich von 40 bis 60 % liegt.

27. Geflochtene, gewebte oder gestrickte Textilie nach einem der Ansprüche 24 bis 26, **dadurch gekennzeichnet, dass** sie in der Form einer linearen, flächigen oder röhrenartigen Textilie vorliegt.

28. Geflochtene Textilie nach einem der Ansprüche 24 bis 27, **dadurch gekennzeichnet, dass** sie mindestens 8 Garne enthält, die jeweils mindestens eine innenliegende füllende Faser umfassen.

29. Geflochtene Textilie nach Anspruch 28, **dadurch gekennzeichnet, dass** der Durchmesser der füllenden Faser im Bereich von 0,0001 bis 1 mm, vorzugsweise im Bereich von 0,01 bis 0,1 mm liegt.

30. Geflochtene Textilie nach Anspruch 28 oder Anspruch 29, **dadurch gekennzeichnet, dass** die füllende Faser von einem Polymer gebildet ist, das aus einer Gruppe ausgewählt ist, die Hyaluronsäure oder deren Derivate, Polymilchsäure, Polyglykolsäure, ein Kopolymer der Polymilchsäure und Polyglykolsäure, Polycaprolacton , Polydioxanon, Polyhydroxyalkanoate, Polyethylen, Polypropylen, Polyetherester, Polyamid oder Polyester, vorzugsweise Polymilchsäure oder ein Kopolymer der Polymilchsäure und Polyglykolsäure, umfasst.

31. Geflochtene Textilie nach einem der Ansprüche 24 bis 30, **dadurch gekennzeichnet, dass** der Flechtwinkel mindestens 20°, vorzugsweise mindestens 25° beträgt, wobei es gilt, dass wenn die Feinheit des Garns x tex beträgt, dann wird bei dem Garn eine Flechtdichte von y cm⁻¹, vorzugsweise von z cm⁻¹ erreicht, wie sich aus der nachfolgenden Tabelle ergibt:
| x | y | z |
|---|---|---|
| Weniger als 20 | 15 | 20 |
| 20 bis 30 | 12 | 15 |
| 30 bis 50 | 9 | 12 |
| 50 bis 80 | 7 | 9 |
| 80 bis 120 | 6 | 7 |
| 120 bis 180 | 5 | 6 |
| 180 bis 270 | 4 | 5 |
| Mehr als 270 | 3 | 4 |

32. Gewebte Textilie nach einem der Ansprüche 24 bis 27, **dadurch gekennzeichnet, dass** wenn die Feinheit des Garns a tex beträgt, dann wird bei dem Gewebe eine Schussdichte von mindestens b cm⁻¹, vorzugsweise von mindestens c cm⁻¹ erreicht, wie sich aus der nachfolgenden Tabelle ergibt:
| a | b | c |
|---|---|---|
| Weniger als 20 | 25 | 35 |
| 20 bis 30 | 18 | 25 |
| 30 bis 50 | 14 | 20 |
| 50 bis 80 | 11 | 16 |
| 80 bis 120 | 9 | 13 |
| 120 bis 180 | 7 | 11 |
| 180 bis 270 | 6 | 9 |
| Mehr als 270 | 5 | 7 |

33. Gestrickte Textilie nach einem der Ansprüche 24 bis 27, **dadurch gekennzeichnet, dass** wenn die Feinheit des Garns e tex beträgt, dann wird bei dem Gestrick eine Maschenstäbchendichte von mindestens f dm⁻¹, vorzugsweise von mindestens g dm⁻¹ erreicht, wie sich aus der nachfolgenden Tabelle ergibt:
| e | f | g |
|---|---|---|
| Weniger als 20 | 20 | 27 |
| 20 bis 30 | 18 | 23 |
| 30 bis 50 | 16 | 20 |
| 50 bis 100 | 14 | 18 |
| Mehr als 270 | 12 | 16 |

34. Anwendung einer geflochtenen, gewebten oder gestrickten Textilie nach einem der Ansprüche 24 bis 33 und einer ungewebten Textilie nach Anspruch 18 auf den Gebieten der Medizin und Gewebetechnik sowie in den Systemen zur kontrollierten Freisetzung.

## Revendications

1. Fibre continue de type âme-gaine à base d'hyaluronane ou d'un dérivé acylé C₁₁-C₁₈ de celui-ci, **caractérisée en ce qu'**elle comprend une combinaison d'hyaluronane et d'un dérivé acylé de celui-ci ou une combinaison de dérivés acylés de celui-ci, l'âme de la fibre et la gaine étant dans un quelconque des agencements suivants:
A) L'âme contient de l'acide hyaluronique ou du sel de celui-ci, la gaine contient de l'hyaluronane acylé C₁₁-C₁₈ ;
B) L'âme contient de l'hyaluronane acylé C₁₁-C₁₈, la gaine contient de l'acide hyaluronique ou du sel de celui-ci ;
C) L'âme contient de l'hyaluronane acylé C₁₁-C₁₈, la gaine contient de l'hyaluronane acylé C₁₁-C₁₈ différent, qui diffère de l'hyaluronane acylé C₁₁-C₁₈ de l'âme par un acyle C₁₁-C₁₈, le degré de substitution d'hyaluronane acylé C₁₁-C₁₈ dans la gaine étant identique ou différent du degré de substitution d'hyaluronane acylé C₁₁-C₁₈ dans l'âme ; ou
D) L'âme contient de l'hyaluronane acylé C₁₁-C₁₈, la gaine contient de l'hyaluronane acylé C₁₁-C₁₈ différent, qui diffère par le degré de substitution d'hyaluronane acylé C₁₁-C₁₈ de l'âme.

2. Fibre selon la revendication 1, **caractérisée en ce que** de l'hyaluronane acylé C₁₁-C₁₈ est acylé dans l'alcool primaire de *N*-acétyle-glucosamine.

3. Fibre selon la revendication 1 ou la revendication 2, **caractérisée en ce que** la masse moléculaire d'hyaluronane acylé C₁₁-C₁₈ est dans la plage de 1 x 10⁵ à 7 x 10⁵ g/mol, de préférence de 2 x 10⁵ à 3 x 10⁵ g/mol.

4. Fibre selon l'une quelconque des revendications précédentes, **caractérisée en ce que** le degré de substitution d'hyaluronane acylé C₁₁-C₁₈ de l'agencement A ou B est dans la plage de 5 à 80 %, de préférence de 30 à 60 %.

5. Fibre selon l'une quelconque des revendications 1 à 3, **caractérisée en ce que** le degré de substitution d'hyaluronane acylé C₁₁-C₁₈, contenu dans l'âme de l'agencement C, est dans la plage de 5 à 80 %, de préférence de 5 à 29 %, mieux encore de 10 à 20 %, et le degré de substitution d'hyaluronane acylé C₁₁-C₁₈ contenu dans la gaine de l'agencement C est dans la plage de 5 à 80 %, de préférence dans la plage de 30 à 80 %, mieux encore de 40 à 60 % ; ou le degré de substitution d'hyaluronane acylé C₁₁-C₁₈ contenu dans l'âme de l'agencement D est dans la plage de 5 à 29 %, de préférence de 10 à 20 % et le degré de substitution d'hyaluronane acylé C₁₁-C₁₈ contenu dans la gaine de l'agencement D est dans la plage de 30 à 80 %, de préférence dans la plage de 40 à 60 %.

6. Fibre selon l'une quelconque des revendications précédentes, **caractérisée en ce que** l'hyaluronane acylé C₁₁-C₁₈ est sélectionné dans le groupe contenant palmitoyle hyaluronane, stéaroyle hyaluronane ou oléoyle hyaluronane.

7. Fibre selon l'une quelconque des revendications 1 à 4 ou selon la revendication 6, **caractérisée en ce que** l'acide hyaluronique et/ou le sel de celui-ci a une masse moléculaire dans la plage de 1 x 10⁵ à 2 x 10⁶ g/mol, de préférence de 8 x 10⁵ à 1.8 x 10⁶ g/mol, le sel d'acide hyaluronique étant sélectionné dans le groupe contenant des ions de métal alcalin, des ions de métal alcalino-terreux, de préférence Na⁺, K⁺, Ca²⁺ ou Mg²⁺.

8. Fibre selon l'une quelconque des revendications précédentes, **caractérisée en ce que** la finesse des fibres est dans la plage de 10 à 40 tex, de préférence de 15 à 35 tex, mieux encore de 22 à 28 tex.

9. Fibre selon l'une quelconque des revendications précédentes, **caractérisée en ce que** le rapport volumique âme:gaine est dans la plage de 3:1 à 1:6, de préférence de 1:3 à 1:5, mieux encore 1:4.

10. Fibre selon l'une quelconque des revendications précédentes, **caractérisée en ce qu'**elle contient au moins un agent actif.

11. Fibre selon la revendication 10, **caractérisée en ce que** l'âme ainsi que la gaine contient au moins un agent actif identique ou différent.

12. Fibre selon la revendication 10 ou la revendication 11, **caractérisée en ce que** l'agent actif est sélectionné dans le groupe, contenant des agents antibactériens, antiseptiques, antibiotiques, anti-inflammatoires, hémostatiques, des anesthésiques, des cytostatiques, des hormones, des immunomodulateurs, des immunosuppresseurs ou des agents pour l'imagerie de contraste, de préférence des nanoparticules magnétiques.

13. Fibre, définie dans l'une quelconque des revendications 1 à 12 pour l'utilisation dans la fabrication des fils, des textiles tressés, tissés, tricotés ou non-tissés.

14. Procédé de préparation des fibres définies dans l'une quelconque des revendications 1 à 13, **caractérisé en ce que** soit pour la préparation de la fibre de l'agencement A ou B, la première solution de filage contenant de l'acide hyaluronique ou du sel de celui-ci à une concentration dans la plage de 1 à 8 % en masse est préparée séparément dans de l'eau et la seconde solution de filage contenant de l'hyaluronane acylé C₁₁-C₁₈ à une concentration de 2 à 8 % en masse est préparée dans un mélange de l'eau et de l'alcool inférieur, où la teneur de l'eau est dans la plage de 30 % (v/v) à 90 % (v/v), la teneur de l'alcool inférieur est dans la plage de 10 % (v/v) à 70 % (v/v), soit pour la préparation de l'agencement C ou D la première solution de filage contenant de l'hyaluronane acylé C₁₁-C₁₈ à une concentration dans la plage de 2 à 8 % en masse est préparée séparément dans un mélange de l'eau et de l'alcool inférieur et la seconde solution de filage contenant de l'hyaluronane acylé C₁₁-C₁₈ différent à une concentration de 2 à 8 % en masse est préparée dans un mélange de l'eau et de l'alcool inférieur, où la teneur de l'eau dans les deux solutions est dans la plage de 30 % (v/v) à 90 % (v/v), la teneur de l'alcool inférieur étant dans la plage de 10 % (v/v) à 70 % (v/v),
après quoi la première solution et la seconde solution de filage sont extrudé ensemble dans un bain de coagulation contenant de 2 à 40 % en masse d'acide organique, de préférence l'acide lactique, au moins 50 % en masse d'alcool inférieur, de préférence d'éthanol, de propan-1-ol ou de propan-2-ol, et de 2 à 48 % en masse d'eau, ce qui résulte en fibre de l'agencement A ou B ou C ou D, qui est en conséquence nettoyée avec de l'alcool inférieur et séchée.

15. Procédé selon la revendication 14, **caractérisé en ce que** la première solution de filage et/ou la seconde solution de filage contient un agent actif.

16. Procédé selon la revendication 14, **caractérisé en ce que** la première solution de filage et/ou la seconde solution de filage contient une composition nano-micellaire à base d'hyaluronane acylé contenant l'agent actif.

17. Fibres discontinues, faites à partir des fibres définies dans l'une quelconque des revendications 1 à 13.

18. Textile non-tissé fait à partir des fibres discontinues définies dans la revendication 17.

19. Fil fait à partir d'au moins une fibre définie dans l'une quelconque des revendications 1 à 13.

20. Fil selon la revendication 19, **caractérisé en ce qu'il** est formé d'un faisceau de fibres contenant 2 à 10 fibres, de préférence 3 à 6.

21. Fil selon la revendication 20, **caractérisé en ce qu'**il est en forme d'un faisceau torsadé de fibres.

22. Fil formé d'au moins deux fibres, **caractérisé en ce qu'au** moins une fibre est la fibre définie dans l'une quelconque des revendications 1 à 13, et au moins une fibre est la fibre, sélectionné dans le groupe des fibres d'autres matériaux biodégradables, les matériaux biodégradable étant sélectionnés dans le groupe comprenant l'acide polylactique, l'acide polyglycolique, un copolymère d'acide polylactique et d'acide polyglycolique, polycaprolactone, polydioxanone ou polyhydroxyalcanoates, de préférence d'acide polylactique ou un copolymère d'acide polylactique et d'acide polyglycolique.

23. Fil selon l'une quelconque des revendications 19 à 22, **caractérisé en ce qu'**il présente une finesse dans la plage de 10 à 400 tex, de préférence 30 à 100 tex.

24. Textile tressé, un textile tissé ou un textile tricoté, **caractérisé en ce qu'**il contient au moins un fil défini dans les revendications 19 à 23.

25. Textile tressé, un textile tissé ou un textile tricoté selon la revendication 24, **caractérisé en ce qu'**il est fait à partir du fil défini dans une quelconque des revendications 19 à 23.

26. Textile tressé, tissé ou tricoté selon la revendication 24 ou la revendication 25, **caractérisé en ce que** l'âme de fibre et la gaine dans le fil sont dans l'agencement A, C ou D, considérant que dans le cas de l'agencement A le degré de substitution de l'hyaluronane acylé C₁₁-C₁₈ dans la gaine est dans la plage de 30 à 80 %, de préférence 40 v 60 %, et dans le cas de l'agencement C ou D le degré de substitution de l'hyaluronane acylé C₁₁-C₁₈ dans l'âme est dans la plage de 5 à 29 %, de préférence 10 à 20 % et le degré de substitution de l'hyaluronane acylé C₁₁-C₁₈ dans la gaine est dans la plage de 30 à 80 %, de préférence 40 à 60 %.

27. Textile tressé, tissé ou tricoté selon l'une quelconque des revendications 24 à 26, **caractérisé en ce qu'**il est en forme de textile linéaire, plane ou tubulaire.

28. Textile tressé selon l'une quelconque des revendications 24 à 27, **caractérisé en ce qu'**il contient au moins 8 fils, contenant au moins une fibre de remplissage dans le textile.

29. Textile tressé selon la revendication 28, **caractérisé en ce que** le diamètre de la fibre de remplissage est dans la plage de 0.0001 à 1 mm, de préférence 0.01 à 0.1 mm.

30. Textile tressé selon la revendication 28 ou la revendication 29, **caractérisé en ce que** la fibre de remplissage est formé d'un polymère sélectionné dans le groupe contenant l'acide hyaluronique ou les dérivés de celui-ci, l'acide polylactique, l'acide polyglycolique, un copolymère d'acide polylactique et d'acide polyglycolique, polycaprolactone, polydioxanone, polyhydroxyalcanoates, polyéthylène, polypropylène, polyétherester, polyamide ou polyester, de préférence l'acide polylactique ou un copolymère d'acide polylactique et d'acide polyglycolique.

31. Textile tressé selon l'une quelconque des revendications 24 à 30, **caractérisé en ce que** l'angle de tressage est au moins 20°, de préférence au moins 25°, alors qu'il s'applique que si la finesse du fil est x tex, la densité de tressage du fil est donc au moins y cm⁻¹, de préférence z cm⁻¹, comme indiqué dans la table ci-dessous
| x | y | z |
|---|---|---|
| Moins de 20 | 15 | 20 |
| 20 à 30 | 12 | 15 |
| 30 à 50 | 9 | 12 |
| 50 à 80 | 7 | 9 |
| 80 à 120 | 6 | 7 |
| 120 à 180 | 5 | 6 |
| 180 à 270 | 4 | 5 |
| Plus de 270 | 3 | 4 |

32. Textile tissé selon l'une quelconque des revendications 24 à 27, **caractérisé en ce que** si la finesse du fil est a tex, le duitage du tissu est donc au moins b cm⁻¹, de préférence au moins c cm⁻¹, comme indiqué dans la table ci-dessous
| a | b | c |
|---|---|---|
| Moins de 20 | 25 | 35 |
| 20 à 30 | 18 | 25 |
| 30 à 50 | 14 | 20 |
| 50 à 80 | 11 | 16 |
| 80 à 120 | 9 | 13 |
| 120 à 180 | 7 | 11 |
| 180 à 270 | 6 | 9 |
| Plus de 270 | 5 | 7 |

33. Textile tricoté selon l'une quelconque des revendications 24 à 27, **caractérisé en ce que** si la finesse du fil est e tex, la densité des colonnes de mailles est donc au moins f dm⁻¹, de préférence au moins g dm⁻¹, comme indiqué dans la table ci-dessous
| e | f | g |
|---|---|---|
| Moins de 20 | 20 | 27 |
| 20 à 30 | 18 | 23 |
| 30 à 50 | 16 | 20 |
| 50 à 100 | 14 | 18 |
| Plus de 270 | 12 | 16 |

34. Utilisation du textile tressé, tissé ou tricoté selon l'une quelconque des revendications 24 à 33 et d'un textile non-tissé selon la revendication 18 dans le domaine de la médecine, de l'ingénierie tissulaire et des systèmes pour la libération contrôlée.
